# EUROPEAN PATENT SPECIFICATION

(11) **EP 3 873 330 B1**
(45) Date of publication and mention of the grant of the patent: **26.06.2024**
(21) Application number: 19877901.9
(22) Date of filing: 31.10.2019
(51) Int. Cl.: H04L 67/12

(54) **SENSOR NETWORK FOR MEASURING PHYSIOLOGICAL PARAMETERS OF MAMMAL SUBJECT AND APPLICATIONS OF SAME**
SENSORNETZWERK ZUR MESSUNG PHYSIOLOGISCHER PARAMETER VON SÄUGETIEREN UND ANWENDUNGEN DAVON
RÉSEAU DE CAPTEURS POUR MESURER DES PARAMÈTRES PHYSIOLOGIQUES D'UN SUJET MAMMIFÈRE ET SES APPLICATIONS

(30) Priority: 31.10.2018 US 201862753625 P; 31.10.2018 US 201862753303 P; 31.10.2018 US 201862753453 P
(43) Date of publication of application: 08.09.2021
(73) Proprietor: Northwestern University, Evanston, IL 60208 (US)
(72) Inventor: ROGERS, John, A., Wilmette, IL 60091 (US); XU, Shuai, Bala Cynwyd, PA 19004 (US); CHUNG, Ha, Uk, Evanston, IL 60208 (US); LEE, Kun, Hyuck, Evanston, IL 60208 (US); HOURLIER-FARGETTE, Aurelie, Evanston, IL 60208 (US); RWEI, Alina, Evanston, IL 60208 (US); LEE, Jong, Yoon, Evanston, IL 60208 (US)
(74) Representative: Weickmann & Weickmann PartmbB
(86) International application number: PCT/US2019/059156
(87) International publication number: WO 2020/092764

(56) References cited:
- US-A1- 2008 036 617
- US-A1- 2009 081 951
- US-A1- 2014 275 888

## Description

Some references, which may include patents, patent applications and various publications, are cited and discussed in the description of this invention. The citation and/or discussion of such references is provided merely to clarify the description of the present invention and is not an admission that any such reference is "prior art" to the invention described herein. All references cited and discussed in this specification are incorporated herein by reference in their entireties and to the same extent as if each reference was individually incorporated by reference.

### FIELD OF THE INVENTION

The invention relates generally to healthcare, and more particularly, to sensor network for measuring physiological parameters of mammal subject and applications of the same.

### BACKGROUND OF THE INVENTION

The background description provided herein is for the purpose of generally presenting the context of the invention. The subject matter discussed in the background of the invention section should not be assumed to be prior art merely as a result of its mention in the background of the invention section. Similarly, a problem mentioned in the background of the invention section or associated with the subject matter of the background of the invention section should not be assumed to have been previously recognized in the prior art. The subject matter in the background of the invention section merely represents different approaches, which in and of themselves may also be inventions. Work of the presently named inventors, to the extent it is described in the background of the invention section, as well as aspects of the description that may not otherwise qualify as prior art at the time of filing, are neither expressly nor impliedly admitted as prior art against the invention.

Every year, 300,000 neonates are admitted to the neonatal care unit (NICU) in the U.S. The Global Fetal and Neonatal Care Equipment market is expected to grow from $7.32 billion in 2016 to reach $11.86 billion by 2022. Vital sign monitoring systems, however, have largely remained locked in time since the 1970s. Large base units with extensive wires are still attached to numerous electrodes.

Continuous monitoring of vital signs in the NICU is essential to the survival of critically-ill neonates. Conventional medical platforms in the NICU fail, however, to offer a safe, patient-centric mode of operation, largely due to the use of hard-wired, rigid interfaces to the neonate's fragile, under-developed skin. Thus, continuous monitoring of vital signs for critical care applications in maternal/fetal and neonatal health requires new technology able to meet unique demands. Small yet adaptable form factors are needed with low skin-device interface stressors. Higher noise to signal ratios require specialized processing and algorithms able to faithfully collect vital signs even during periods of high motion artifact.

Therefore, a heretofore unaddressed need exists in the art to address the aforementioned deficiencies and inadequacies.

### SUMMARY OF THE INVENTION

One aspect of the invention is to provide a new class of wireless wearable sensors capable of re-capitulating standard of care monitoring systems, with relevance for deployment in pediatric health, in a highly networked and synchronized fashion. These wireless wearable sensors are in a networked configuration to facilitate real time data processing, analytics, and safety features that meet the rigorous demands of clinical care. The sensor network has advanced monitoring capabilities and greater safety features, and is applicable to both low-resource settings where wireless vital sign monitoring systems have not penetrated into neonatal care as well as high-resource settings where the instant systems represent the cutting edge, next generation systems for neonatal monitoring.

In one aspect, the invention relates to a sensor network for measuring physiological parameters of a mammal subject. The physiological parameters include, but are not limited to, one or more of heart activities including a stroke volume and ejection fraction, oxygenation level, temperature, skin temperature differentials, body movement, body position, breathing parameters, blood pressure, crying time, crying frequency, swallow count, swallow frequency, chest wall displacement, heart sounds, core body position, asynchronous limb motion, speaking, and biomechanical perturbation. The mammal subject can be a living human subject or a living non-human subject. In certain embodiments, physiological parameters of neonates or infants are monitored and measured. It should be appreciated to one skilled in the art that physiological parameters of children or adults can also be monitored and measured in practice the invention.

In one embodiment, the sensor network includes a plurality of spatially separated sensor systems that is time-synchronized to each other. Each of the plurality of spatially separated sensor systems is attached to a respective position of the mammal subject and includes a sensor member for measuring at least one physiological parameter, a system on a chip (SoC) having a microprocessor coupled to the sensor member for receiving data from the sensor member and processing the received data, and a transceiver coupled to the SoC for wireless data transmission and wireless power harvesting. The sensor network also includes a microcontroller unit (MCU) adapted in wireless communication with the plurality of spatially separated sensor systems for wirelessly transmitting data to and from the plurality of spatially separated sensor systems.

In one embodiment, each two adjacent sensor systems are spatially separated by a respective distance that is adjustable between a minimal distance and a maximal distance.

In one embodiment, the plurality of spatially separated sensor systems comprises a first sensor system configured to attach to a central region of the mammal subject and a second sensor system configured to attach to an extremity region of the mammal subject. In one embodiment, the central region comprises one or more of a chest region, a neck region including a suprasternal notch area, and a head region including a forehead region or an anterior fontanelle region of the mammal subject. The extremity region comprises one or more of a limb region, a foot region, a hand region, a toenail region, and a fingernail region of the mammal subject.

In one embodiment, the sensor member of the first sensor system comprises at least two electrodes spatially apart from each other for electrocardiogram (ECG) generation.

In one embodiment, the sensor member of the second sensor system comprises a photoplethysmogram (PPG) sensor comprising an optical source and an optical detector located within a sensor footprint.

In one embodiment, each sensor member of the first sensor system and the second sensor system further comprises one or more of an accelerometer for measuring at least one of a position and a movement; an inertial measurement unit (IMU) for measuring at least one of a movement, a force, an angular rate, and an orientation; and a temperature sensor for measuring temperature.

In one embodiment, the accelerometer or the IMU is used to measure at least one of seismocardiography (SCG) and a respiratory rate.

In one embodiment, the accelerometer or the IMU is used with a motion artifact module to identify a vital sign as subject to motion artifact and to correct of motion artifact.

In one embodiment, each sensor member of the first sensor system and the second sensor system comprises one or more of an ECG sensor, an EKG sensor, a pulse oximeter sensor, a temperature sensor, a blood pressure sensor, an accelerometer, or an acoustic sensor.

In one embodiment, the MCU is configured to perform at least one function of receiving and processing measured data of the physiological parameters from the plurality of spatially separated sensor systems; transmitting the processed data of the physiological parameters to at least one of a patient database, a cloud server, and a mobile device; continuously multi-modal monitoring one or more critical parameters associating at least one vital sign; and notifying a practitioner or caregiver when a sensor aberrant signal output condition occurs; and generating an alarm when an alarming vital sign reading condition in which the one or more of the critical parameters are out of pre-defined ranges occurs, and notifying a practitioner or caregiver of the alarm. In one embodiment, the one or more critical parameters are one or more of the heart parameters, brain activities, temperature, body movements, respiratory parameters, oxygenation, vocalization parameters, swallow parameters, and blood pressure and blood flow.

In one embodiment, the MCU is configured to further perform at least one function of assessing body pain of the mammal subject, based on the physiological parameters including a heart rate, heart rate variability, respiratory rate, respiratory effort, and crying time, and notifying a practitioner or caregiver where the pain is assessed; assessing local blood perfusion of the mammal subject, based on a location specific pulse oximetry derived from peripheral oxygen saturation (SpO₂) measured by pulse oximeters of the sensor systems placed on various locations of the body; detecting an apneic event when sudden decreases or cessation of the respiratory rate followed by compensatory increases in the heart rate and decreases in the SpO₂, and notifying a practitioner or caregiver when the apneic event occurs, and vibrating the sensor systems itself to trigger the mammal subject to change its position or awake from sleep; and localizing anatomical pathology based on the physiological parameters measured by the plurality of spatially separated sensor systems placed in differential locations.

In one embodiment, the MCU comprises a mobile device for at least one of real-time display of the physiological parameters, recording of the physiological parameters, and alarm.

In one embodiment, the mobile device in bi-directionally wireless communication with the plurality of spatially separated sensor systems. In one embodiment, the mobile device is in wireless communication with the patient database. In one embodiment, the mobile device is a hand-held device or a portable device having a graphical user interface to display the plurality of physiological parameters.

In one embodiment, the sensor network further comprises a power unit for wirelessly powering the sensor systems. In one embodiment, the sensor network is capable of operating wirelessly for a period of at least 24 hours without a source of external power.

In one embodiment, at least one of the plurality of spatially separated sensor systems further comprises an actuator configured to generate a force for providing a stimulus to the mammal subject when a pre-defined trigger signal is detected. In one embodiment, the stimulus comprises gentle vibrations for soothing the mammal subject.

In one embodiment, the actuator is one or more of an electromechanical motor, a heater, and an electrical stimulator.

In one embodiment, each of the plurality of spatially separated sensor systems further comprises a plurality of flexible and stretchable interconnects electrically connecting to a plurality of electronic components including the sensor member, the SoC and the transceiver; and an elastomeric encapsulation layer surrounding the electronic components and the plurality of flexible and stretchable interconnects to form a tissue-facing surface and an environment-facing surface, wherein the tissue-facing surface is configured to conform to a skin surface of the mammal subject. In one embodiment, the encapsulation layer comprises a flame retardant material.

In one embodiment, the transceiver comprises a magnetic loop antenna configured to allow simultaneous wireless data transmission and wireless power harvesting through a single link.

In one embodiment, the electronic components of each of the plurality of spatially separated sensor systems further comprises a battery for provide power to said sensor system, and the elastomeric encapsulation layer is configured to electrically isolate the battery from the mammal subject during use. In one embodiment, the battery is a rechargeable battery operably recharged with wireless recharging.

In one embodiment, the electronic components of each of the plurality of spatially separated sensor systems further comprises a failure prevention element that is a short-circuit protection component or a battery circuit to avoid battery explosion.

In another aspect, the invention relates to a method for measuring physiological parameters of a mammal subject. In one embodiment, the method includes deploying the sensor network as disclosed above; synchronizing the plurality of spatially separated sensor systems to a common time base; measuring data of the physiological parameters from the plurality of spatially separated sensor systems; and wirelessly transmitting the time synchronized measured physiological parameters. Said deploying the sensor network including respectively attaching at least one first sensor system to a central region of the mammal subject and at least one second sensor system to an extremity region of the mammal subject such that the first sensor system and the second sensor system are spatially separated by a distance.

In one embodiment, said wirelessly transmitting the time synchronized measured physiological parameters is performed with the MCU in wireless communication with the plurality of spatially separated sensor systems.

In one embodiment, said wirelessly transmitting the time synchronized measured physiological parameters comprises receiving and processing the measured data of the physiological parameters from the plurality of spatially separated sensor systems; and transmitting the processed data of the physiological parameters to at least one of a patient database, a cloud server, and a mobile device.

In one embodiment, the method further comprises associating the wirelessly transmitted physiological parameters with a unique patient identifier, thereby identifying the physiological parameters with the mammal subject in at least one of the patient database, the cloud server, and the mobile device.

In one embodiment, the method further comprises identifying a normal vital sign reading condition, an aberrant sensor output condition, or an alarming vital sign reading condition, based on the physiological parameters. In one embodiment, the aberrant sensor output condition is one or more of a lead off state where the tissue-facing surface of a sensor system is not in intimate contact with an underlying patient surface; a patient motion artifact; or an output discrepancy relative to at least two different sensor systems. In one embodiment, the aberrant sensor output condition is identified when at least one vital sign detected from the first sensor system differs from that detected from the second sensor by 25% or greater.

In one embodiment, the method further comprises continuously multi-modal monitoring one or more critical parameters associating with the at least one vital sign; and notifying a practitioner or caregiver when the sensor aberrant signal output condition occurs.

In one embodiment, the method further comprises generating an alarm when the alarming vital sign reading condition in which the one or more of the critical parameters are out of pre-defined ranges occurs, and notifying a practitioner or caregiver of the alarm.

In one embodiment, the one or more critical parameters are one or more of the heart parameters, brain activities, temperature, body movements, respiratory parameters, oxygenation, vocalization parameters, swallow parameters, and blood pressure and blood flow.

In one embodiment, the method further comprises at least one of assessing body pain of the mammal subject, based on the physiological parameters including a heart rate, heart rate variability, respiratory rate, respiratory effort, and crying time, and notifying a practitioner or caregiver where the pain is assessed; assessing local blood perfusion of the mammal subject, based on a location specific pulse oximetry derived from the SpO₂ measured by pulse oximeters of the sensor systems placed on various locations of the body; detecting an apneic event when sudden decreases or cessation of the respiratory rate followed by compensatory increases in the heart rate and decreases in the SpO₂, and notifying a practitioner or caregiver when the apneic event occurs, and vibrating the sensor systems itself to trigger the mammal subject to change its position or awake from sleep; and localizing anatomical pathology based on the physiological parameters measured by the plurality of spatially separated sensor systems placed in differential locations.

In one embodiment, the method further comprises displaying at least one of the physiological parameters, notifications, and the alarm in a display having a graphical user interface.

In one embodiment, the method further comprises securing the display of the physiological parameters by requiring a secure login before a user can access the physiological parameters.

In one embodiment, the user is a practitioner or caregiver, the mammal subject or family member, and the method further comprises wirelessly communicating between the practitioner or caregiver and the mammal subject or family member based on the physiological parameters.

In one embodiment, the method further comprises securely sending a command from a remote practitioner or caregiver to a practitioner or caregiver in proximity to the mammal subject based on the physiological parameters.

In one embodiment, the method further comprises actuating an actuator to generate a force for providing a stimulus to the mammal subject when a pre-defined trigger signal is detected. In one embodiment, the stimulus comprises gentle vibrations for soothing the mammal subj ect.

In yet another aspect, the invention relates to a non-transitory tangible computer-readable medium storing instructions which, when executed by one or more processors, cause the above-disclosed method to be performed.

### BRIEF DESCRIPTION OF THE DRAWINGS

The following drawings form part of the present specification and are included to further demonstrate certain aspects of the invention. The invention may be better understood by reference to one or more of these drawings in combination with the detailed description of specific embodiments presented herein. The drawings described below are for illustration purposes only. The drawings are not intended to limit the scope of the present teachings in any way.
FIG. 1A schematically shows a functional block diagram of a sensor network according to certain embodiments of the present invention.
FIG. 1B schematically shows a deployment of a sensor network according to certain embodiments of the present invention. The sensor system in the upper left panel is configured to attach to a torso region, such as the chest, while the sensor system in the upper right panel is configured to attach to an appendage, such as a foot, as illustrated in the bottom left and bottom right panels, respectively.

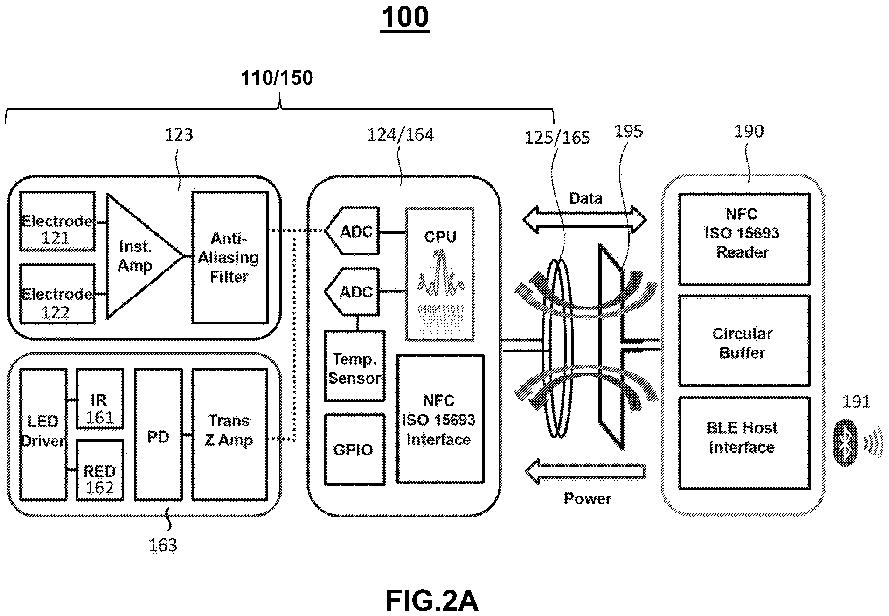

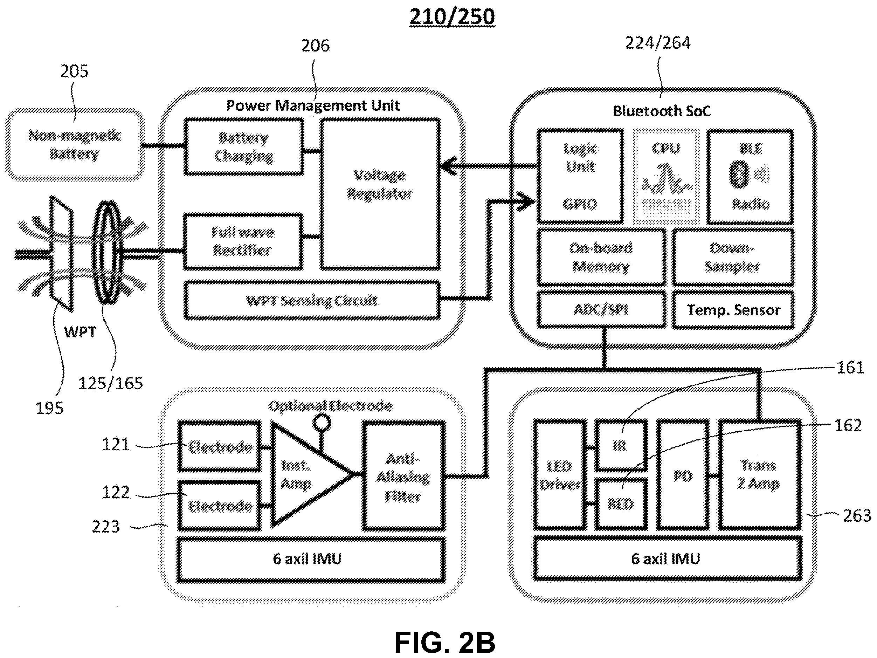

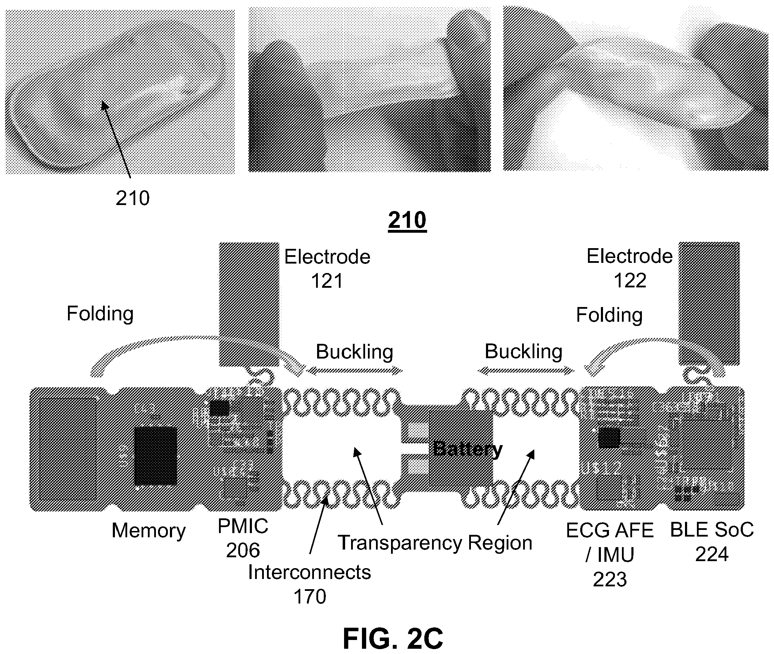

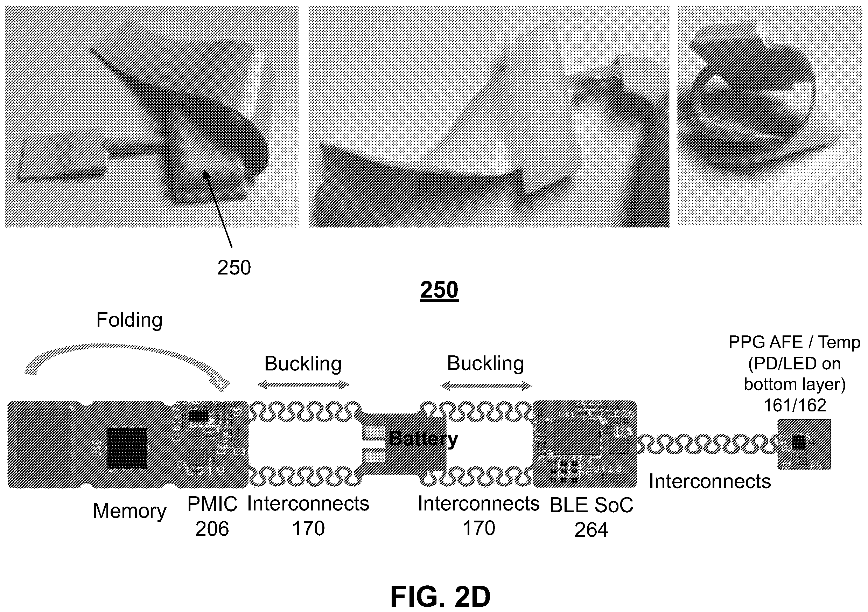

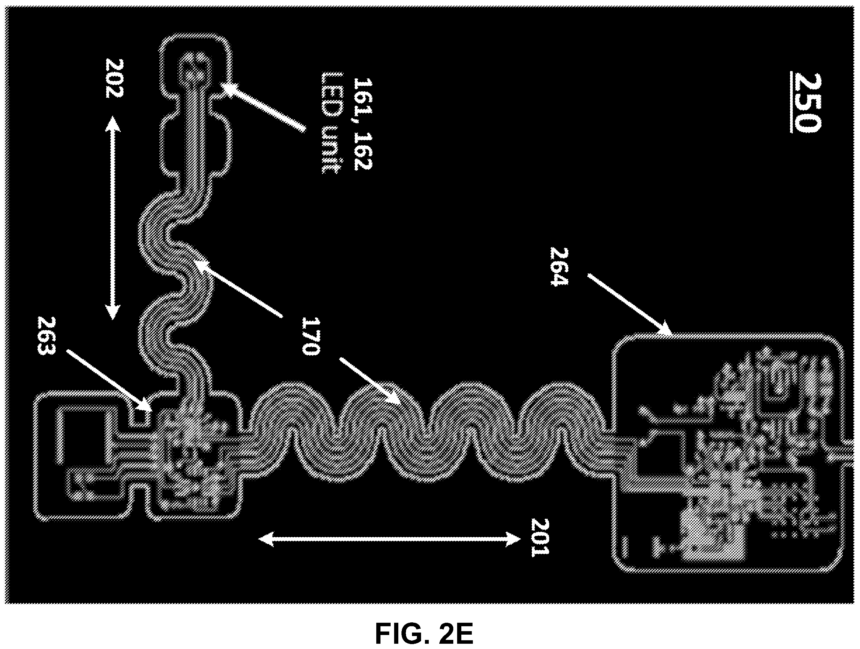

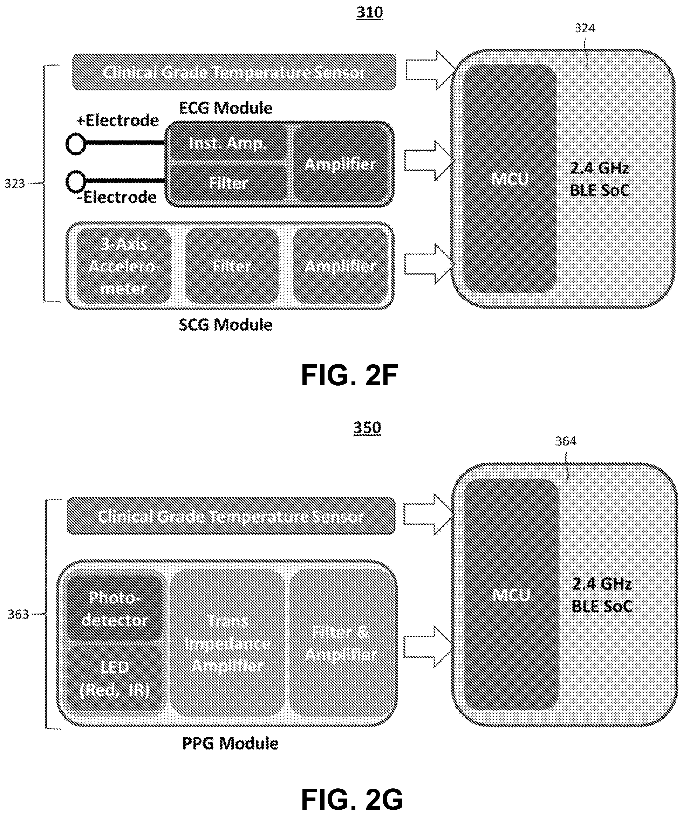

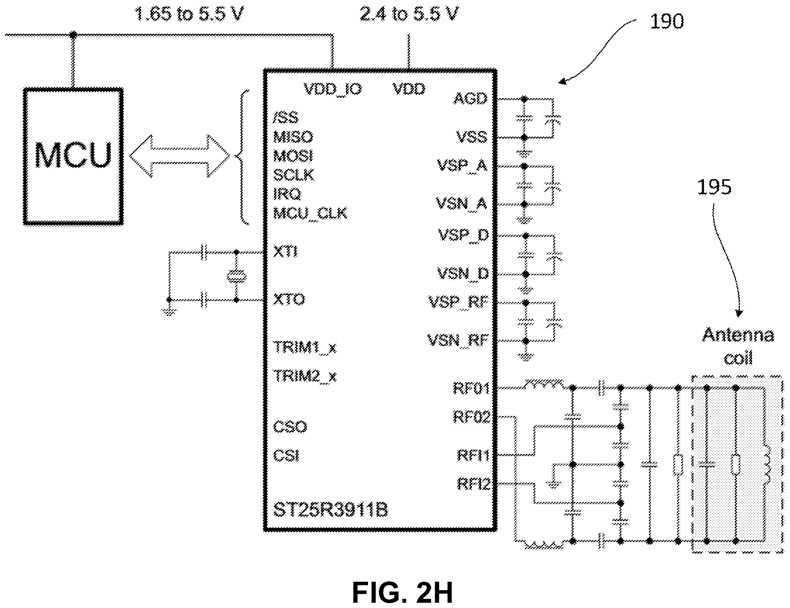

FIGS. 2A-2H show schematic illustrations of ultra-thin, skin-like wireless sensor systems used in a sensor network, according to embodiments of the invention. FIG. 2A is a functional block diagram showing analog front end and electronic components of each EES, components of the near-field communication (NFC) system on a chip (SoC) including microcontroller, general-purpose input/output (GPIO), and radio interface, with a host reader platform that includes an NFC reader module and a Bluetooth low energy (BLE) interface with circular buffer. FIG. 2B shows a functional block diagram of the two sensor systems according to another embodiment of the invention. The power management unit involves dual power operation mode from primary wireless power transfer and the secondary battery for portability. The ECG EES includes optional electrode for fECG measurement and 6 axial inertial measurement unit (IMU) for seismocardiography (SCG) and respiratory rate measurement on top of ECG analog front end. The PPG EES includes the pulse oximetry analog front end and 6 axial IMU for motion artifact reduction algorithm. Each individual unit is controlled by BLE SoC. FIG. 2C is a schematic of a sensor system configured to attach to the torso, such as a chest, according to one embodiment of the invention. The sensor system is stretchable and foldable, as illustrated in the top panels. The electrical components and layout are illustrated in the bottom panel, including the components providing a networked and wireless platform (including a power unit, a memory unit, a temperature unit, an ECG unit, a BLE SoC). FIG. 2D shows a sensor system configured to attach on an extremity, such as a foot, leg, hand, arm finger, toe or nail, such as by a wrapping-type mechanism to secure the main circuit components with a mechanically decoupled sensor system connected thereto, according to one embodiment of the invention. The sensor system is stretchable and foldable, as illustrated in the top panels. The electrical components (including power unit, memory unit, temperature unit, PPG sensor, Bluetooth low energy (BLE)) and layout are illustrated in the bottom panel, including the components to provide a networked and wireless platform. The sensor portion may be aligned in a different direction, such as an orthogonal direction relative to the main circuit components. In this manner, the main circuit components may wrap around the foot, with the sensor portion independently mountable, such as to a nail region. In one embodiment, the PPG sensor system for measuring oxygenation with a sensor having an LED unit. The main circuit component, including a microprocessor and a wireless transmitter are aligned in one direction, referred to as a wrap direction. The LED unit extends at a different direction from the wrap direction. In this manner, the main circuit component can be wrapped around an extremity without any adhesive, such as around an ankle. The active sensor portion can independently be attached to a desired location, such as a foot or a toenail. In this manner, only a small footprint of the total device needs to be reliably affixed to the patient, and can even be affixed with an adhesive to a surface that can more readily withstand the adhesive and corresponding removal, such as a nail. FIG. 2E is a schematic representation of PPG device for measuring oxygenation with a sensor having an LED unit, according to one embodiment of the invention. The main circuit component, including a microprocessor and a wireless transmitter are aligned in one direction, referred to as a wrap direction. The LED unit extends at a different direction from the wrap direction. In this manner, the main circuit component can be wrapped around an extremity without any adhesive, such as around an ankle. The active sensor portion can independently be mounted at a desired location, such as a foot or a toenail. In this manner, only a small footprint of the total device needs to be reliably affixed to the patient, and can even be affixed with an adhesive to a surface that can more readily withstand the adhesive and corresponding removal, such as a nail. FIG. 2F is a schematic diagram of an ECG sensor system that measures electric potential, temperature, position and motion that are wirelessly transmitted to a microprocessor, illustrated as part of an MCU, according to one embodiment of the invention. FIG. 2G is a schematic diagram of a PPG sensor system to measure oxygen and that measures also temperature, that are transmitted to a microprocessor, illustrated as part of an MCU, according to one embodiment of the invention. FIG. 2H shows an exemplary NFC Reader IC, illustrating MCU, IC and antenna, according to one embodiment of the invention.
FIG. 3 is an illustration of two sensor systems (chest unit and foot unit) of a sensor network that support continuous 24/7 use without external wires and, at most, a gentle adhesive such as a hydrogel, according to one embodiment of the invention.
FIG. 4 shows a PPG sensor system configured to conformally wrap around an appendage (to panel), with the sensor wrapped around a doll and a neonate foot in the respective bottom panels, according to one embodiment of the invention.
FIG. 5 shows a flowchart of a method of monitoring physiological parameters of a mammal subject according to certain embodiments of the invention.
FIG. 6 shows schematically a flowchart of the onboard software operation of the sensors of the sensor network according to certain embodiments of the invention.
FIG. 7 shows schematically an illustration of general sensing, storage and telemedical applications of a sensor network according to certain embodiments of the invention.
FIGS. 8A-8C illustrate the use of an ECG sensor for respiratory rate (RR) estimation according to certain embodiments of the invention. FIG. 8A shows an algorithm using an ECG sensor for respiratory rate (RR) estimation. FIG. 8B illustrates the RR estimation using the algorithm shown in FIG. 8A, based on the ECG signal measured by the ECG sensor. FIG. 8C shows a plot of the actual count compared to the ECG EES.
FIGS. 9A-9B illustrates the use of chest wall movements, measured by an accelerometer, for RR estimation according to certain embodiments of the invention. FIG. 9A shows an algorithm using chest wall movements for the RR estimation. FIG. 9B illustrates the RR estimation using the algorithm shown in FIG. 9A, based on the chest wall movements measured by the accelerometer.
FIG. 10 shows signal processing algorithms of continuous wavelet transform (CWT) DST for pulse oximetry according to certain embodiments of the invention.
FIG. 11A and 11B illustrate SpO₂ determination for a clean signal (no motion artifact) and a noisy signal (motion contaminated signal), respectively, according to certain embodiments of the invention.
FIG. 12 shows that, for heart rate, the mean difference from the experimental system compared to standard-of-care system was -0.47 beats per minute aggregated across 18 subjects, according to certain embodiments of the invention. The standard deviation of the mean difference was 3.0 beats per minute. Thus, 95% of the data fall within a discrepancy less than 6.0 beats per minute. The agreement of the experimental wireless system remained comparable at lower heart rates (<100 beats per minute) and higher heart rates (>170 beats per minute).

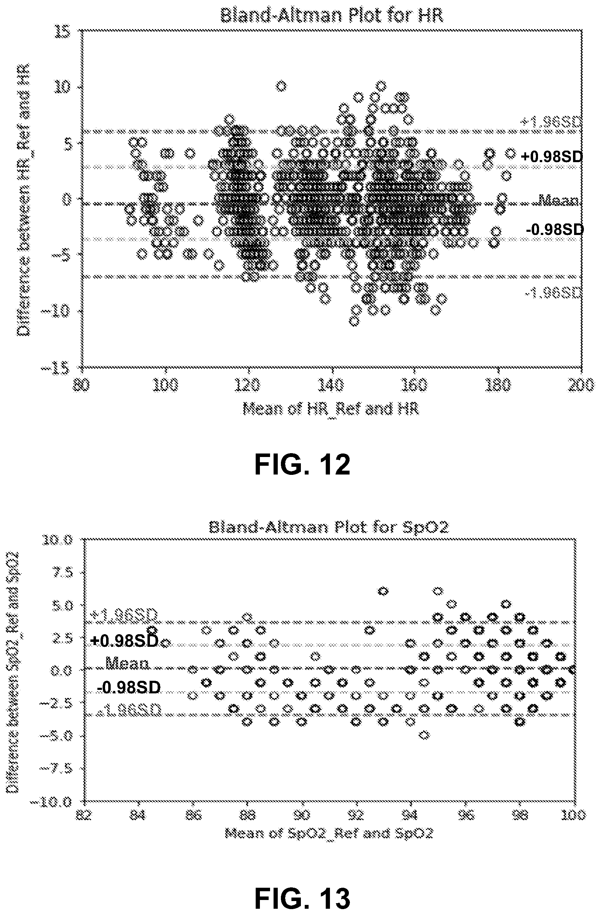

FIG. 13 shows that, for blood oxygenation, the mean difference was 0.11% and the standard deviation of the mean difference was 1.8% between the experimental system and the standard-of-care system aggregated across 16 subjects, according to certain embodiments of the invention. Thus, 95% of the data fall within a discrepancy less than 3.6% blood oxygenation. The agreement of the experimental wireless system remained comparable at both lower blood oxygenation (<90%) as well as higher blood oxygenation (>96%). Two subjects had intravenous lines or other medically necessary equipment on both lower extremities and excluded.

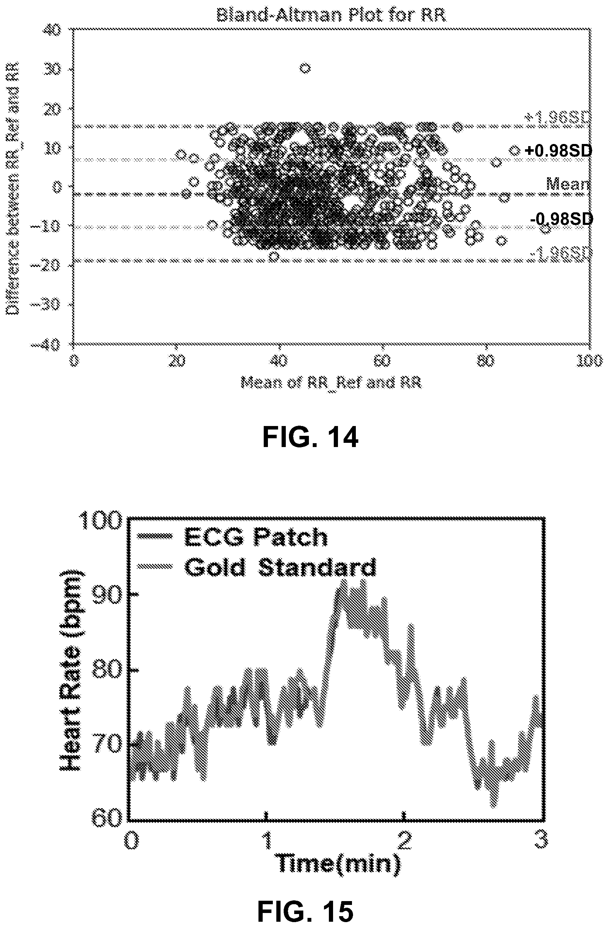

FIG. 14 shows that, for respiratory rate, the mean differences was -2.0 breaths per minute with a standard deviation of mean differences of 8.7 breaths per minute between the experimental system and the reference system according to certain embodiments of the invention. The higher variation for respiratory rate may relate to differences in the measuring modality of respiratory rate. Respiratory rate derived by the experimental system is through the ECG while the reference Phillips monitor utilizes thoracic impedance pneumography). Similar performance is noted for measurements across low and high respiration rates (<30 and >70 breaths per minute).
FIG. 15 shows the heart rate determined by a sensor network according to certain embodiments of the invention, illustrating the equivalent performance to an FDA-approved device.
FIG. 16 shows comparisons of a PPG sensor compared to a gold-standard system, according to certain embodiments of the invention.
FIG. 17 shows a neonatal forehead measurement for SpO₂ measurements, according to certain embodiments of the invention.
FIG. 18A are photographs of skin surface before placement (top panels) and for sensor placement (bottom panels) for chest attached (left panels) and foot attached (right panels), according to certain embodiments of the invention.
FIG. 18B shows skin after removal (top panels) and 30 minutes after removal (bottom panels), according to certain embodiments of the invention.
FIG. 19 shows thermal images of the chest region after 24 hours of sensor mounting immediately before (top panel) and after removal (bottom panel) revealing no heating of the skin and no heat generation, according to certain embodiments of the invention.
FIG. 20 illustrates different temperature sensor locations in a sensor network according to certain embodiments of the invention.
FIG. 21 is a graph of temperature calibration curves for a chest (top panel) and foot (bottom panel) unit according to certain embodiments of the invention.
FIG. 22 illustrates good agreement and correlation with the instant temperature sensors and a gold standard temperature sensor, according to certain embodiments of the invention.
FIG. 23 illustrates the differences in temperature measurement for the sensors illustrated in FIG. 21 compared to core temperature (top panel) and compared to infrared temperature measurement (bottom panel) according to certain embodiments of the invention.
FIG. 24 is a graphical representation of three-dimensional sensor position (x, y, z) for a sleeping, held in arms and held against body (kangaroo mother care or "KMC"), according to certain embodiments of the invention.
FIG. 25 shows the average (x, y, z) locations determined by a sensor system having an accelerometer, for an infant in different positions reflected by the photographs, according to certain embodiments of the invention.
FIG. 26 is a graphical representation of the location results for a sleeping, feeding, holding and KMC position according to certain embodiments of the invention.
FIG. 27 is a summary of the measure (x, y, z) positions according to certain embodiments of the invention.
FIG. 28 is an accelerometer output for burping of a neonate in a clinical study according to certain embodiments of the invention.
FIG. 29 is a summary of an in-lab simulation showing x, y, z postion over time, with a KMC and tilt maneuver, according to certain embodiments of the invention.
FIGS. 30A-30B summarize the KMC accelerometer sensor measurement and processing, respectively, according to certain embodiments of the invention. Various learning algorithms, such as supervised and unsupervised learning, assist in obtaining and classifying the data.
FIG. 31 illustrates data labeling for posture, tilt and movement based on an accelerometer sensor system, according to certain embodiments of the invention.
FIGS. 32A-32B and 33-38 illustrate the powerful use of a remote receiver, where a GUI can provide easy to understand monitoring to remote caregiver or family member, according to certain embodiments of the invention.
FIG. 39 shows a flowchart summarizing various telemedical applications of the sensor network system, according to certain embodiments of the invention. Specifically, the sensor can be viewed by both the neonate's caregivers as well as their providers. The availability of skilled neonatologists trained in critical care is limited in developing countries or rural communities. The wearable sensors in this disclosure can also enable secure physician/clinician view allowing for eNICU care at a distance.

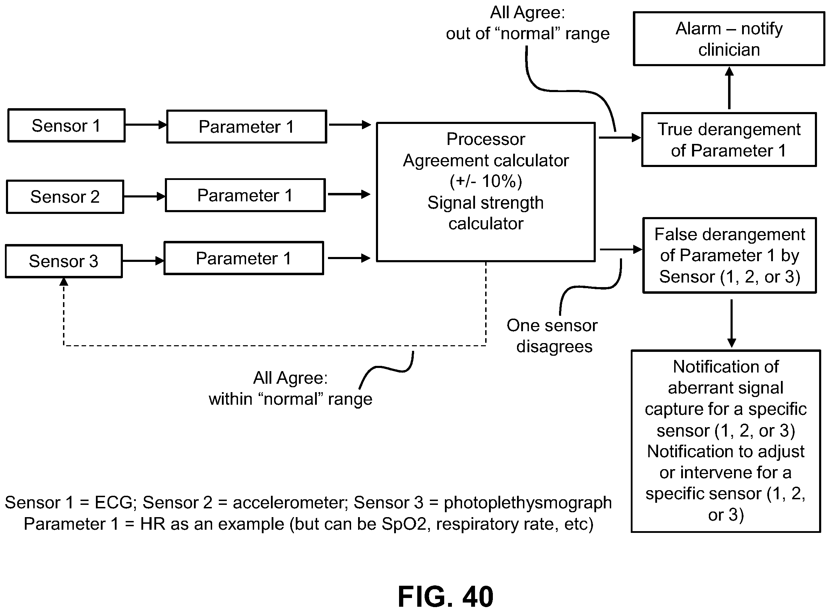

FIG. 40 is a flowchart summary illustrating the benefits of measuring a physiological parameter with at least three different sensors according to certain embodiments of the invention. If all sensors obtain a similar reading that is out of a specified normal range for the parameter, a caregiver is notified. If one sensor is out of a defined agreement range (e.g., +/-10%, or a user-determined range that may be relevant for the specific sensors being used and vital sign being measure), a notification is sent of an aberrant signal for that sensor so that remedial action can be taken. Alternatively, if all sensors are in agreement that the parameter is outside a "normal" range, the clinician may be notified so that immediate intervention/assessment is made. Of course, even while this is occurring, the sensors may be continuously in operation providing parameter measurements. For normal operating conditions, the dashed arrows reflect the sensor network simply continues normal operation.
FIG. 41 is a flowchart summary similar to FIG. 40, but with an additional Sensor 4 accelerometer that is used to compensate and correct for motion artifacts, thereby further increasing sensor accuracy and reliability.
FIG. 42 is a schematic illustration of the wireless communication and a Bluetooth time synchronization to provide time-synchronized sensors, according to certain embodiments of the invention.

### DETAILED DESCRIPTION OF THE INVENTION

The present invention will now be described more fully hereinafter with reference to the accompanying drawings, in which exemplary embodiments of the present invention are shown. The present invention may, however, be embodied in many different forms and should not be construed as limited to the embodiments set forth herein. Rather, these embodiments are provided so that this disclosure will be thorough and complete, and will fully convey the scope of the invention to those skilled in the art. Like reference numerals refer to like elements throughout.

The terms used in this specification generally have their ordinary meanings in the art, within the context of the invention, and in the specific context where each term is used. Certain terms that are used to describe the invention are discussed below, or elsewhere in the specification, to provide additional guidance to the practitioner regarding the description of the invention. For convenience, certain terms may be highlighted, for example using italics and/or quotation marks. The use of highlighting and/or capital letters has no influence on the scope and meaning of a term; the scope and meaning of a term are the same, in the same context, whether or not it is highlighted and/or in capital letters. It will be appreciated that the same thing can be said in more than one way. Consequently, alternative language and synonyms may be used for any one or more of the terms discussed herein, nor is any special significance to be placed upon whether or not a term is elaborated or discussed herein. Synonyms for certain terms are provided. A recital of one or more synonyms does not exclude the use of other synonyms. The use of examples anywhere in this specification, including examples of any terms discussed herein, is illustrative only and in no way limits the scope and meaning of the invention or of any exemplified term. Likewise, the invention is not limited to various embodiments given in this specification.

It will be understood that, although the terms first, second, third, etc. may be used herein to describe various elements, components, regions, layers and/or sections, these elements, components, regions, layers and/or sections should not be limited by these terms. These terms are only used to distinguish one element, component, region, layer or section from another element, component, region, layer or section. Thus, a first element, component, region, layer or section discussed below can be termed a second element, component, region, layer or section without departing from the teachings of the present invention.

It will be understood that, as used in the description herein and throughout the claims that follow, the meaning of "a", "an", and "the" includes plural reference unless the context clearly dictates otherwise. Also, it will be understood that when an element is referred to as being "on," "attached" to, "connected" to, "coupled" with, "contacting," etc., another element, it can be directly on, attached to, connected to, coupled with or contacting the other element or intervening elements may also be present. In contrast, when an element is referred to as being, for example, "directly on," "directly attached" to, "directly connected" to, "directly coupled" with or "directly contacting" another element, there are no intervening elements present. It will also be appreciated by those of skill in the art that references to a structure or feature that is disposed "adjacent" to another feature may have portions that overlap or underlie the adjacent feature.

It will be further understood that the terms "comprises" and/or "comprising," or "includes" and/or "including" or "has" and/or "having" when used in this specification specify the presence of stated features, regions, integers, steps, operations, elements, and/or components, but do not preclude the presence or addition of one or more other features, regions, integers, steps, operations, elements, components, and/or groups thereof.

Furthermore, relative terms, such as "lower" or "bottom" and "upper" or "top," may be used herein to describe one element's relationship to another element as illustrated in the figures. It will be understood that relative terms are intended to encompass different orientations of the device in addition to the orientation shown in the figures. For example, if the device in one of the figures is turned over, elements described as being on the "lower" side of other elements would then be oriented on the "upper" sides of the other elements. The exemplary term "lower" can, therefore, encompass both an orientation of lower and upper, depending on the particular orientation of the figure. Similarly, if the device in one of the figures is turned over, elements described as "below" or "beneath" other elements would then be oriented "above" the other elements. The exemplary terms "below" or "beneath" can, therefore, encompass both an orientation of above and below.

Unless otherwise defined, all terms (including technical and scientific terms) used herein have the same meaning as commonly understood by one of ordinary skill in the art to which the present invention belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

As used in this disclosure, "around", "about", "approximately" or "substantially" shall generally mean within 20 percent, preferably within 10 percent, and more preferably within 5 percent of a given value or range. Numerical quantities given herein are approximate, meaning that the term "around", "about", "approximately" or "substantially" can be inferred if not expressly stated.

As used in this disclosure, the phrase "at least one of A, B, and C" should be construed to mean a logical (A or B or C), using a non-exclusive logical OR. As used herein, the term "and/or" includes any and all combinations of one or more of the associated listed items.

As used in this disclosure, the term "spatially separated" refers to two different locations on skin, wherein the two sensor systems disposed on those locations are not in physical contact. For example, one sensor system may be on the torso, and another sensor system on the foot.

As used in this disclosure, the term "mammal subject" refers to a living human subject or a living non-human subject. For the purpose of illustration of the invention, the apparatus and method are applied to monitor and/or measure physiological parameters of neonates or infants. It should be appreciated to one skilled in the art that the apparatus can also be applied to monitor and/or measure physiological parameters of children or adults in practice the invention.

The description below is merely illustrative in nature and is in no way intended to limit the invention, its application, or uses. The broad teachings of the invention can be implemented in a variety of forms. Therefore, while this invention includes particular examples, the true scope of the invention should not be so limited since other modifications will become apparent upon a study of the drawings, the specification, and the following claims. For purposes of clarity, the same reference numbers will be used in the drawings to identify similar elements. It should be understood that one or more steps within a method may be executed in different order (or concurrently) without altering the principles of the invention.

To address the aforementioned deficiencies and inadequacies, the invention in one aspect discloses a new class of wireless wearable sensors capable of re-capitulating standard of care monitoring systems, with relevance for deployment in pediatric health, in a highly networked and synchronized fashion. These wireless wearable sensors are in a networked configuration to facilitate real time data processing, analytics, and safety features that meet the rigorous demands of clinical care. The sensor network has advanced monitoring capabilities and greater safety features, and is applicable to both low-resource settings where wireless vital sign monitoring systems have not penetrated into neonatal care as well as high-resource settings where the instant systems represent the cutting edge, next generation systems for neonatal monitoring.

In one aspect, the invention relates to a sensor network for or non-invasively and continuously measuring physiological parameters of a mammal subject. Physiological parameters that can be measured include, but are not limited to, one or more of heart activities including a stroke volume and ejection fraction, oxygenation level, temperature, skin temperature differentials, body movement, body position, breathing parameters, ballistocardiography, respiratory effort, blood pressure, crying time, crying frequency, swallow count, swallow frequency, chest wall displacement, heart sounds, core body position, asynchronous limb motion, speaking, and biomechanical perturbation.

In certain embodiments, the sensor network includes a plurality of spatially separated sensor systems that is time-synchronized to each other. Each of the plurality of spatially separated sensor systems is attached to a respective position of the mammal subject and includes a sensor member for measuring at least one physiological parameter, a system on a chip (SoC) having a microprocessor coupled to the sensor member for receiving data from the sensor member and processing the received data, and a transceiver coupled to the SoC for wireless data transmission and wireless power harvesting. The sensor network also includes a microcontroller unit (MCU) adapted in bidirectional wireless communication with the plurality of spatially separated sensor systems for wirelessly transmitting data to and from the plurality of spatially separated sensor systems.

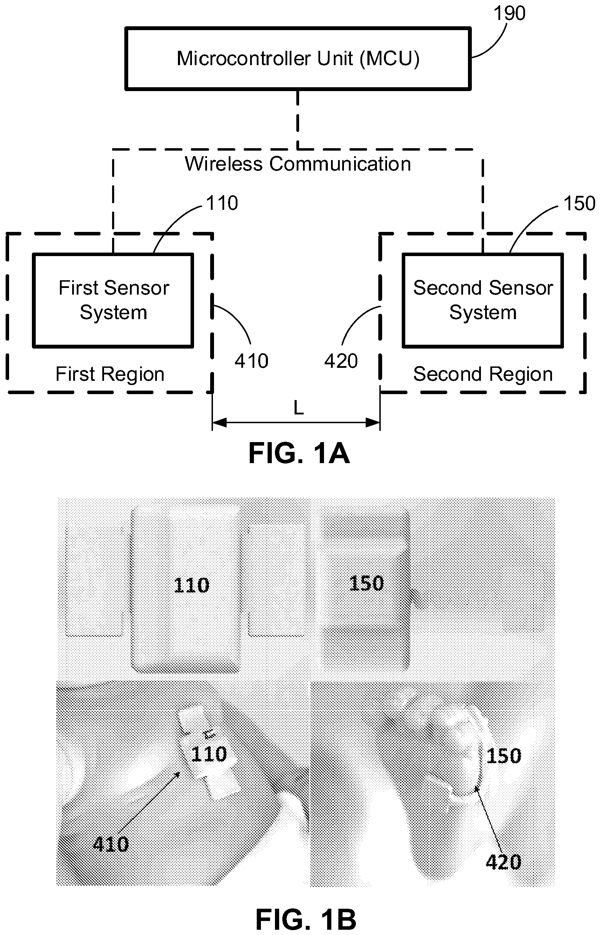

FIG. 1A schematically shows a functional block diagram of a sensor network according to one embodiment of the present invention. In the exemplary embodiments, the sensor network 100 includes first sensor system(s) 110 and second sensor system(s) 150 that are time-synchronized to each other, and a microcontroller unit (MCU) (alternatively, a reader system) 190 adapted in wireless communication with the first sensor system 110 and the second sensor system 150. In certain embodiments, each of the first sensor system 110 and the second sensor system 150 is in wireless communication with the MCU 190 via a wireless transmission protocol, such as a near field communication (NFC) protocol, or Bluetooth protocol. Specifically, the term "time-synchronized" (or "time-synced") refers to measurement of a parameter by different sensors, at different locations, that are synchronized in time to allow for measurement of novel physiological parameters.

In certain embodiments, each of the first sensor system 110 and the second sensor system 150 is an epidermal electronic system (EES). For example, the first sensor system 110 is configured to attach to a central region, while the second sensor system 150 is configured to attach to an extremity region of the mammal subject. The central region includes one or more of a chest region, a neck region including a suprasternal notch area, and a head region including a forehead region or an anterior fontanelle region of the mammal subject. The extremity region includes one or more of a limb region, a foot region, a hand region, a toenail region, and a fingernail region of the mammal subject.

In certain embodiments, the first sensor system 110 is a conformable torso sensor system configured to attach and conform to a torso region of the mammal subject for recording electrocardiogram (ECG) data and skin temperature; and the second sensor system 150 is a conformable extremity sensor system configured to attach and conform to a limb or appendage region of the mammal subject for recording photoplethysmogram (PPG) data and skin temperature. In other words, the first sensor system 110 can be an electrocardiography (ECG), and the second sensor system 150 can be a PPG sensor system. As shown in FIGS. 1A and 1B, the torso sensor system 110 and the extremity sensor system 150 are operably attached to a first region 410, which is, for example, a torso region such as the chest, and a second region 420, which is, for example, a limb region 420 such as a foot, respectively, of the neonate so that the torso sensor system 110 and the extremity sensor system 150 are spatially separated by a distance L. The distance L between the torso sensor system 110 and the extremity sensor system 150 is adjustable between a minimal distance and a maximal distance.

In certain embodiments, each of the torso sensor system 110 and the extremity sensor system 150 includes a sensor member (circuit) having one or more sensors that are used to detect a vital sign of the mammal subject, and then to generate one or more corresponding physiological parameters, an SoC having a microprocessor coupled to the sensor member (circuit) for receiving data from the sensor member and processing the received data, and a transceiver coupled to the SoC for wireless data transmission and wireless power harvesting. The sensors may be various types of sensors for detecting the vital sign as a signal, and the signal can be, for example, an electrical signal related to at least one of ECG and electromyography (EMG) technology; a mechanical signal related to movement, respiration and arterial tonometry; an acoustic signal related to vocal cord vocalization and heart sound; and an optical signal related to blood oxygenation, and so on. In certain embodiments, each sensor member of the first sensor system 110 and the second sensor system 150 comprises one or more of an ECG sensor, an EKG sensor, a pulse oximeter sensor, a temperature sensor, a blood pressure sensor, an accelerometer, and an acoustic sensor.

Referring to FIG. 2A, in one embodiment, the sensor circuit (member) 123 of the torso sensor system 110 includes, but is not limited to, two electrodes 121 and 122 spatially separated from each other for ECG generation. The electrodes 121 and 122 can be either mesh electrodes or solid electrodes. The sensor member 123 also includes, but is not limited to, an instrumentation amplifier (e.g., Inst. Amp) electrically coupled to the two electrodes 121 and 122, adapted for eliminating the need for input impedance matching and thus making the amplifier particularly suitable for use in measurement and test equipment, and an anti-aliasing filter (AAF) electrically couple to the instrumentation amplifier and used before a signal sampler to restrict the bandwidth of a signal to approximately or completely satisfy the Nyquist-Shannon sampling theorem over the band of interest.

Still referring to FIG. 2A, the SoC 124 of the torso sensor system 110 includes, but is not limited to, a microprocessor unit, e.g., CPU, a near-field communication (NFC) interface, e.g., NFC ISO 15693 interface, general-purpose input/output (GPIO) ports, one or more temperature sensors (Temp. sensor), and analog-to-digital converters (ADCs) in communication with each other, for receiving data from the sensor member 123 and processing the received data.

Also referring to FIG. 2A, the transceiver 125 of the torso sensor system 110 is coupled to the SoC 124 for wireless data transmission and wireless power harvesting. In the exemplary embodiment, the transceiver 125 comprises a magnetic loop antenna tuned to compliance with the NFC protocol and configured to allow simultaneous wireless data transmission and wireless power harvesting through a single link.

As shown in FIG. 2A, the sensor member (circuit) 163 of the extremity sensor system 150 includes a PPG sensor located within a sensor footprint, which has an optical source having an infrared (IR) light emitting diode (LED) 161 and a red LED 162, and an optical detector (PD) electrically coupled to the IR LED 161 and the red LED 162. The sensor member 163 also includes, but is not limited to, an LED driver electrically coupled to the two electrodes 161 and 162 for driving the IR LED 161 and the red LED 162, and a trans Z amplifier electrically coupled to the PD.

Similarly, as shown in FIG. 2A, the SoC 164 of the extremity sensor system 150 includes, but is not limited to, a microprocessor unit, e.g., CPU, a near-field communication (NFC) interface, e.g., NFC ISO 15693 interface, general-purpose input/output (GPIO) ports, one or more temperature sensors (Temp. sensor), and analog-to-digital converters (ADCs) in communication with each other, for receiving data from the sensor member (circuit) 163 and processing the received data. The transceiver 165 of the extremity sensor system 150 is coupled to the SoC 164 for wireless data transmission and wireless power harvesting. In the exemplary embodiment, the transceiver 165 comprises a magnetic loop antenna tuned to compliance with the NFC protocol and configured to allow simultaneous wireless data transmission and wireless power harvesting through a single link.

In addition, each of the plurality of spatially separated sensor systems further comprises a plurality of flexible and stretchable interconnects (FIGS. 2C-2D) electrically connecting to a plurality of electronic components including the sensor member, the SoC and the transceiver; and an elastomeric encapsulation layer (FIGS. 2C-2D) surrounding the electronic components and the plurality of flexible and stretchable interconnects to form a tissue-facing surface and an environment-facing surface, wherein the tissue-facing surface is configured to conform to a skin surface of the mammal subject. In one embodiment, the encapsulation layer comprises a flame retardant material.

In operation, the torso sensor system 110 and the extremity sensor system 150 are in wireless communication with a reader system 190, alternatively, the MCU, having an antenna 195, where one exemplary integrated circuit (IC) of the NFC reader system 190 is shown in FIG. 2H. Specifically, the RF loop antennas 125 and 165 in both the torso sensor system 110 and the extremity sensor system 150 are in wireless communication with the antenna 195 and serve dual purposes in power transfer and in data communication, as shown in FIG. 2A. In one embodiment, the reader system 190 also includes, but is not limited to, an NFC ISO 15693 reader, a circular buffer and a Bluetooth Low Energy (BLE) interface, which are configured such that data can be continuously streamed at rates of up to 800 bytes/s with dual channels, which is orders of magnitude higher than those previously achieved in NFC sensors. A key to realizing such high rates is in minimizing the overhead associated with transfer by packaging data into 6 blocks (24 Bytes) in the circular buffer. The antenna 195 connects to the host system for simultaneous transfer of RF power to the torso sensor system 110 and the extremity sensor system 150. As such, the sensor network can operate at vertical distances through biological tissues, bedding, blankets, padded mattresses, wires, sensors and other materials found in NICU incubators, for full coverage wireless operation in a typical incubator. BLE radio transmission then allows transfer of data to a personal computer, tablet computer or smartphone with a range of up to 20 m. Connections to central monitoring systems in the hospital can then be established in a straightforward manner.

In another embodiment as shown in FIGS. 2B-2D, the first sensor system 210 and the second sensor system 250 are similar to the first sensor system 110 and the second sensor system 150 shown in FIG. 1A, except that each of the first sensor system 210 and the second sensor system 250 further comprises a battery 205 for provide power to said sensor system, and a power management unit/IC (PMIC) 206 electrically coupled with the battery 205, the Bluetooth SoC 224/264 and the transceiver (antenna) 195. The power management unit 206 operably involves dual power operation mode from primary wireless power transfer and the secondary battery 205 for portability. In addition, the sensor member (or sensor circuit) 223 of the first sensor system 210 also includes optional electrode for fECG measurement and 6 axial inertial measurement unit (IMU) for seismocardiography (SCG) and respiratory rate measurement on the top of an ECG analog front end (AFE). The sensor member (or sensor circuit) 263 of the second sensor system 250 also includes also a PPG AFE and 6 axial IMU for motion artifact reduction algorithm. The BLE SoC 224/264 of each of the first sensor system 210 and the second sensor system 250 further comprises a down-sampler and BLE radio. Each of the power management unit 206 and the sensor members 223 and 263 is controlled by BLE SoC 224/264. In one embodiment, the accelerometer or the IMU is used to measure at least one of seismocardiography (SCG) and a respiratory rate. In one embodiment, the accelerometer or the IMU is used with a motion artifact module to identify a vital sign as subject to motion artifact and to correct of motion artifact.

In certain embodiments, the battery 205 is a rechargeable battery operably recharged with wireless recharging. In one embodiment, the electronic components of each of the first sensor system 210 and the second sensor system 250 further comprises a failure prevention element that is a short-circuit protection component or a battery circuit (not shown) to avoid battery explosion. In one embodiment, the elastomeric encapsulation layer is configured to electrically isolate the battery from the mammal subject during use.

Additionally, the first sensor system 210 is stretchable and foldable, as illustrated in the top panels of FIG. 2C. The electrical components and layout are illustrated in the bottom panel of FIG. 2C, including the components providing a networked and wireless platform (including a power unit, a memory unit, a temperature unit, an ECG unit, and a BLE SoC).

The second sensor system 250, as shown in FIG. 2D, is configured to attach on an extremity, such as a foot, leg, hand, arm finger, toe or nail, such as by a wrapping-type mechanism to secure the main circuit components with a mechanically decoupled sensor system connected thereto. The second sensor system 250 is also stretchable and foldable, as illustrated in the top panels of FIG. 2D. The electrical components including a power unit, a memory unit, a temperature unit, an ECG unit, and a BLE SoC and layout are illustrated in the bottom panel of FIG. 2D, including the components to provide a networked and wireless platform. The sensor portion may be aligned in a different direction, such as an orthogonal direction relative to the main circuit components. As such, the main circuit components may wrap around the foot, with the sensor portion independently mountable, such as to a nail region. In one embodiment, the main circuit component can be wrapped around an extremity without any adhesive, such as around an ankle. The active sensor portion can independently be attached to a desired location, such as a foot or a toenail. In this manner, only a small footprint of the total device needs to be reliably affixed to the patient, and can even be affixed with an adhesive to a surface that can more readily withstand the adhesive and corresponding removal, such as a nail.

FIG. 2E shows schematically another spatial arrangement of the main circuit component 264 and the sensor circuit 263 having an LED units 161 and 162 of the PPG EES 250 (or 150) according to one embodiment of the invention. The main circuit component 264 and the sensor circuit 263 including the LED units 161 and 162 are connected by the serpentine, flexible and stretchable interconnects 170. The main circuit component 264 including at least the SoC and the transceiver is aligned and operably wrapped around a limb or appendage in a wrap direction 201. The LED units 161 and 162 extend at a different direction 202 from the wrap direction 201. As such, the main circuit component 264 can be wrapped around an extremity without any adhesive, such as around an ankle. The active sensor portion can independently be mounted at a desired location, such as a foot or a toenail. Accordingly, only a small footprint of the total device needs to be reliably affixed to the patient, and can even be affixed with an adhesive to a surface that can more readily withstand the adhesive and corresponding removal, such as a nail. In one embodiment, the sensor member of the PPG EES 250 (or 150) is conformable to a skin surface and configured as a soft wrap for circumferential attaching to the limb or appendage region.

In yet another embodiment as shown in FIGS. 2F-2G, the first sensor system 310 and the second sensor system 350 are similar to the first sensor system 110 and the second sensor system 150 shown in FIG. 1A, except that each sensor member 323/363 of the first sensor system 310 and the second sensor system 350 further comprises a clinical grade temperature sensor. In addition, the sensor member 323 of the first sensor system 310 also includes 3-axis accelerometer. Furthermore, each of the first sensor system 310 and the second sensor system 350 includes, instead of an NFC SoC 124/164 shown in FIG. 1A, a BLE SoC 324/264.

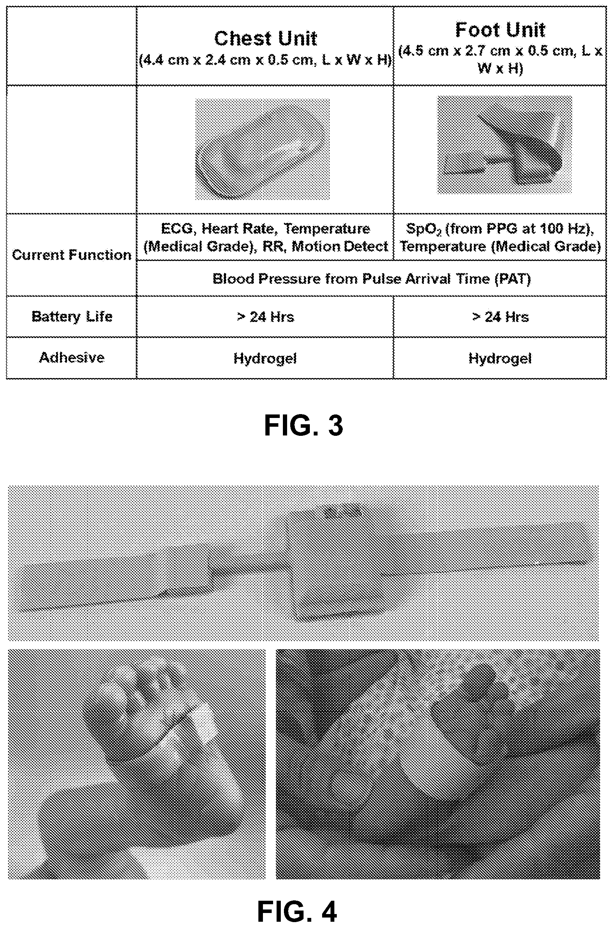

FIG. 3 is an illustration of torso and extremity sensor systems (chest unit and foot unit) of a sensor network that support continuous 24/7 use without external wires and, at most, a gentle adhesive such as a hydrogel, according to one embodiment of the invention. In this exemplary embodiment, the torso sensor system (i.e., Chest Unit) has an L × W × H dimension of 4.4 cm × 2.4 cm × 0.5 cm, and adapted to measure ECG, heart rate, temperature (medical grade), RR, motion detect. the extremity sensor system (i.e., Foot Unit) has an L × W × H dimension of 4.5 cm × 2.7 cm ×0.5 cm, and adapted to measure SpO₂ (from PPG at 100 Hz), and temperature (medical grade).

FIG. 4 shows one embodiment of a PPG sensor system configured to conformally wrap around an appendage (to panel), with the sensor wrapped around a doll and a neonate foot in the respective bottom panels.

Referring back to FIG. 1A, the MCU 190 is configured to receive, from the sensor systems 110 and 150, output signals representing the physiological parameters, and to display the physiological parameters of the mammal subject.

Specifically, in certain embodiments, the MCU 190 is configured to perform at least one function of receiving and processing measured data of the physiological parameters from the plurality of spatially separated sensor systems; transmitting the processed data of the physiological parameters to at least one of a patient database, a cloud server, and a mobile device; continuously multi-modal monitoring one or more critical parameters associating at least one vital sign; and notifying a practitioner or caregiver when a sensor aberrant signal output condition occurs; and generating an alarm when an alarming vital sign reading condition in which the one or more of the critical parameters are out of pre-defined ranges occurs, and notifying a practitioner or caregiver of the alarm. In one embodiment, the one or more critical parameters are one or more of the heart parameters, brain activities, temperature, body movements, respiratory parameters, oxygenation, vocalization parameters, swallow parameters, and blood pressure and blood flow.

In certain embodiments, the MCU 190 is configured to further perform at least one function of assessing body pain of the mammal subject, based on the physiological parameters including a heart rate, heart rate variability, respiratory rate, respiratory effort, and crying time, and notifying a practitioner or caregiver where the pain is assessed; assessing local blood perfusion of the mammal subject, based on a location specific pulse oximetry derived from peripheral oxygen saturation (SpO₂) measured by pulse oximeters of the sensor systems placed on various locations of the body; detecting an apneic event when sudden decreases or cessation of the respiratory rate followed by compensatory increases in the heart rate and decreases in the SpO₂; and notifying a practitioner or caregiver when the apneic event occurs, and vibrating the sensor systems itself to trigger the mammal subject to change its position or awake from sleep; and localizing anatomical pathology based on the physiological parameters measured by the plurality of spatially separated sensor systems placed in differential locations. In one embodiment, the MCU, for instance, can be used to localize lung pathology for the diagnosis of lobe-specific pneumonia or infection.

In one embodiment, the MCU comprises a mobile device for at least one of real-time display of the physiological parameters, recording of the physiological parameters, and alarm.

In one embodiment, the mobile device in bi-directionally wireless communication with the plurality of spatially separated sensor systems. In one embodiment, the mobile device is in wireless communication with the patient database. In one embodiment, the mobile device is a hand-held device or a portable device having a graphical user interface to display the plurality of physiological parameters.

In one embodiment, the sensor network further comprises a power unit for wirelessly powering the sensor systems. In one embodiment, the sensor network is capable of operating wirelessly for a period of at least 24 hours without a source of external power.

In one embodiment, at least one of the plurality of spatially separated sensor systems further comprises an actuator configured to generate a force for providing a stimulus to the mammal subject when a pre-defined trigger signal is detected. In one embodiment, the stimulus comprises gentle vibrations for soothing the mammal subject. In one embodiment, the actuator is one or more of an electromechanical motor, a heater, and an electrical stimulator.

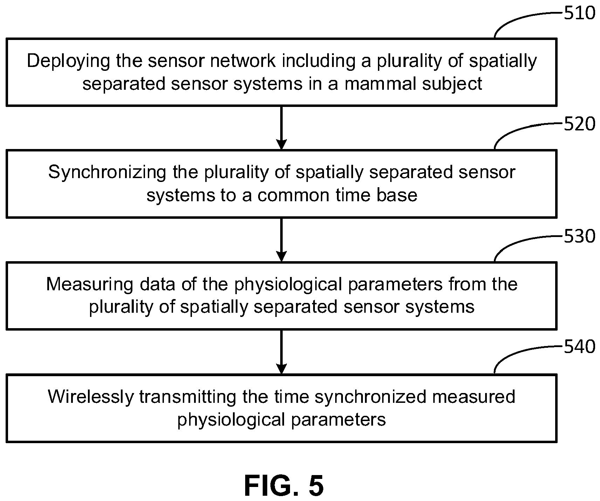

FIG. 5 shows a flowchart of a method for measuring physiological parameters of a mammal subject, according to certain embodiments of the invention. In certain embodiments, the method as shown in FIG. 5 may be implemented on the sensor network shown in FIG. 1A. It should be particularly noted that, unless otherwise stated in the disclosure, the steps of the method may be arranged in a different sequential order, and are thus not limited to the sequential order as shown in FIG. 5.

As shown in FIG. 5, at procedure 510, a sensor network including a plurality of sensor systems (i.e., the first sensor system 110 and the second sensor system 150 as shown in FIGS. 1A1B) are deployed in the mammal subject. Said deploying procedure 510 includes respectively attaching at least one first sensor system to a central region of the mammal subject and at least one second sensor system to an extremity region of the mammal subject such that the first sensor system and the second sensor system are spatially separated by a distance. For example, the first sensor system 110 is attached to a first position in the torso region 410 of the mammal subject for measuring a heartbeat of the mammal subject, and the second sensor system 150 is attached to a second position in the limb region 420 of the mammal subject for measuring a pulse of the mammal subject. Further, the sensor systems 110 and 150 are in wireless communication with the MCU 190, and spatially separated by a distance defined by the first and second positions. As deployed, the sensor network is corresponding to a bidirectional wireless communication system. As used in the disclosure, the term "bidirectional wireless communication system" refers to onboard components of the sensor that provides capability of receiving and sending signals. In this manner, an output may be provided to an external device, including a cloud-based device, personal portable device, or a caregiver's computer system. Similarly, a command may be sent to the sensors, such as by an external controller, which may or may not correspond to the external device. Machine learning algorithms may be employed to improve signal analysis and, in turn, command signals sent to the medical sensor, including a stimulator of the medical sensor for providing haptic signal to a user of the medical device useful in a therapy. More generally, these systems may be incorporated into a processor, such as a microprocessor located on-board or physically remote from the electronic device of the medical sensor.

At procedure 520, the plurality of spatially separated sensor systems is time-synchronized to a common time base. As used in the disclosure, the term "time-synchronized" or "time synced" refers to measurement of a parameter by different sensors, including at different locations, which are synchronized in time to allow for measurement of novel physiological parameters. Examples include master-slave linked sensor systems that allows for time synced measurements. For example, certain embodiments of the invention utilize a multiprotocol functionality that incorporates a secondary 2.4Ghz radio protocol other than Bluetooth to create a private star network among the network of sensors. The secondary radio protocol allows one of the sensors to act as the central hub to broadcast the local clock based on its crystal oscillator to create a common clock within the sensor network. Every sensor can have a local clock running and will adjust the local clock value based on the broadcasted clock value. The central hub can additionally communicate with the base station (including, for example, a remote reader or a receiver) to synchronize its local clock to the base station's clock. The private star network is not bounded to the base station allowing two different body-sensor networks to be synchronized in time without the need for a central hub. This is relevant in situations where the sensors function as blind data collection tools with data that can be downloaded and used later via a base unit.

The common clock can timestamp all of the signals captured through the sensors that the private star network uses allowing novel algorithms that depend on a common clock to be used in the sensor system. The only source of time lag/drift is from the crystal oscillator that is typically low (0.0004%). This time lag can be adjusted and corrected via the central hub at a frequency determined by the user.

At procedure 530, data of the physiological parameters are measured from the plurality of spatially separated sensor systems;

At procedure 540, the time synchronized measured physiological parameters are wirelessly transmitted to at least one of a patient database, a cloud server, and a mobile device. In one embodiment, said wirelessly transmitting procedure 540 includes receiving and processing the measured data of the physiological parameters from the plurality of spatially separated sensor systems; and transmitting the processed data of the physiological parameters to at least one of a patient database, a cloud server, and a mobile device.

In one embodiment, said wirelessly transmitting procedure 540 is performed with the MCU in wireless communication with the plurality of spatially separated sensor systems.

In one embodiment, the method further comprises associating the wirelessly transmitted physiological parameters with a unique patient identifier, thereby identifying the physiological parameters with the mammal subject in at least one of the patient database, the cloud server, and the mobile device.

In one embodiment, the method further comprises identifying a normal vital sign reading condition, an aberrant sensor output condition, or an alarming vital sign reading condition, based on the physiological parameters. In one embodiment, the aberrant sensor output condition is one or more of a lead off state where the tissue-facing surface of a sensor system is not in intimate contact with an underlying patient surface; a patient motion artifact; or an output discrepancy relative to at least two different sensor systems. In one embodiment, the aberrant sensor output condition is identified when at least one vital sign detected from the first sensor system differs from that detected from the second sensor by 25% or greater.

In one embodiment, the method further comprises continuously multi-modal monitoring one or more critical parameters associating with the at least one vital sign; and notifying a practitioner or caregiver when the sensor aberrant signal output condition occurs.

In one embodiment, the method further comprises generating an alarm when the alarming vital sign reading condition in which the one or more of the critical parameters are out of pre-defined ranges occurs, and notifying a practitioner or caregiver of the alarm.

In one embodiment, the one or more critical parameters are one or more of the heart parameters, brain activities, temperature, body movements, respiratory parameters, oxygenation, vocalization parameters, swallow parameters, and blood pressure and blood flow.

In one embodiment, the method further comprises at least one of assessing body pain of the mammal subject, based on the physiological parameters including a heart rate, heart rate variability, respiratory rate, respiratory effort, and crying time, and notifying a practitioner or caregiver where the pain is assessed; assessing local blood perfusion of the mammal subject, based on a location specific pulse oximetry derived from the SpO₂ measured by pulse oximeters of the sensor systems placed on various locations of the body; detecting an apneic event when sudden decreases or cessation of the respiratory rate followed by compensatory increases in the heart rate and decreases in the SpO₂, and notifying a practitioner or caregiver when the apneic event occurs, and vibrating the sensor systems itself to trigger the mammal subject to change its position or awake from sleep; and localizing anatomical pathology based on the physiological parameters measured by the plurality of spatially separated sensor systems placed in differential locations.

In one embodiment, the method further comprises displaying at least one of the physiological parameters, notifications, and the alarm in a display having a graphical user interface.

In one embodiment, the method further comprises securing the display of the physiological parameters by requiring a secure login before a user can access the physiological parameters.

In one embodiment, the user is a practitioner or caregiver, the mammal subject or family member, and the method further comprises wirelessly communicating between the practitioner or caregiver and the mammal subject or family member based on the physiological parameters.

In one embodiment, the method further comprises securely sending a command from a remote practitioner or caregiver to a practitioner or caregiver in proximity to the mammal subject based on the physiological parameters.

In one embodiment, the method further comprises actuating an actuator to generate a force for providing a stimulus to the mammal subject when a pre-defined trigger signal is detected. In one embodiment, the stimulus comprises gentle vibrations for soothing the mammal subj ect.

It should be noted that all or a part of the methods according to the embodiments of the invention is implemented by hardware or a program instructing relevant hardware.

Yet another aspect of the invention provides a non-transitory computer readable storage medium/memory which stores computer executable instructions or program codes. The computer executable instructions or program codes enable a computer or a similar computing apparatus to complete various operations in the above disclosed method of non-invasively measuring physiological parameters of a mammal subject. The storage medium/memory may include, but is not limited to, high-speed random access medium/memory such as DRAM, SRAM, DDR RAM or other random access solid state memory devices, and non-volatile memory such as one or more magnetic disk storage devices, optical disk storage devices, flash memory devices, or other non-volatile solid state storage devices.

According to the invention, the sensor network has applications including, but not limited to, critical care monitoring in neonatal intensive care units; critical care monitoring in pediatric intensive care units; critical care monitoring in neonatal/pediatric cardiac care units; critical care monitoring in neonatal/pediatric neurocritical care units; home monitoring for high-risk neonates.

The sensor networks according to embodiments of the invention have a number of advantages. For example, the sensor systems are fully integrated and capable of recapitulating full vital signs monitoring necessary for critical care. The at least two sensor systems include, but is not limited to, ECG, high-frequency accelerometer, gyroscope, temperature sensor, photoplethysmography unit, EMG. These sensors enable the widest measurement to date for a wearable sensor system that include traditional vital signs and novel physiological metrics that extend beyond current standard of care.

Sensing capabilities include, but are not limited to, vital signs: heart rate, respiratory rate, skin temperature (central and peripheral), core temperature sensor (when placed underneath the axilla), blood oxygenation.

Novel physiological metrics include, but are not limited to, cuff-less blood pressure (via pulse arrival time or pulse transit time), crying time, swallow count, chest wall displacement, heart sounds, core body position, skin temperature differentials between a central location (e.g. trunk) and distal location (e.g. limb).

The sensor network according to embodiments of the invention can be used to correlate chest wall movement overlying the heart with stroke volume and ejection fraction measured by echocardiography.

The sensor network according to embodiments of the invention allows analysis of a baby's position including when a baby is upright. This can be used to quantify important parameters such as how long a baby is being held for kangaroo care.

According to embodiments of the invention, the output of the sensor systems can be used to assess a variety of metrics, including to determine whether a baby has sustained an injury that is consistent with non-accidental trauma (e.g. child abuse), and can measure vocalization, crying time as a metric of discomfort or pain, asynchronous motion of the limbs, and/or sleep quality assessments with core body position measurement (laying face down, on the side, on the back); neonates sleep safest flat on their back, with other positions at a greater risk for sudden infant death syndrome (SIDS).

According to embodiments of the invention, unique mechanical layouts enable safe deployment on the fragile skin of mobile infants. Layered design of the circuitry allows for mechanical isolation of higher modulus components in a mechanical island; bending/twisting/stretching does not disturb the underyling sensor or other stain-sensitive components. The pulse oximeter unit is configured as a soft wrap that allows for circumferential affixation to the foot or hand of a neonate. This configuration reduces motion artifact and allows for maximal signal fidelity for SpO₂. The soft, mechanical nature of the sensor itself enables intimate skin coupling without the need of powerful adhesives reducing the risk for iatrogenic skin injuries. Embodiments where the form factor occupies a small surface area (less than 2.0 cm × 4.0 cm) facilitates placement on a premature neonate.

According to embodiments of the invention, unique isolation of electrical components enable operation in critical care settings. Battery is current isolated allowing for enhanced electrical safety to the neonate. Electrodes are able to be adjusted in spacing - allowing for accommodation of subjects of drastically different chest wall sizes (e.g. premature infant at 25 weeks gestational age vs adult subject). The electrical components are shielded enabling operation even during life-saving medical interventions such as cardiac defibrillation. Low power operation enabling 24 hours of continuous use between charges. Battery has failure preventions including short circuit protection and battery explosion circuitry. Flame retardation of the elastomer encapsulation reduces risk of injury.

According to embodiments of the invention, the wearable sensors fully support Bluetooth 5 technology enabling drastically extended sensing ranges with a base unit. Master-slave linked sensor system allows for time synced measurements of novel physiological parameters. Of course, the network systems provided herein are compatible with any number of communication protocols, including ultra wide band and narrow band communication protocols.

According to embodiments of the invention, the sensor systems provide transparent windows allowing for direct visual inspection of the skin underneath without removing the system. Perforated holes enable wicking away of sweat. Adhesives that have variable peel force based on the directionality of removal. Adhesives are placed strategically around the edges of the device (and not the center) to reduce total skin adhesive area as a method of reducing the risk of iatrogenic injury. Methods where a long-lasting adhesive (>24 hours adherence) is placed on the neonate with an additional mechanical communication. Peel force on sensitive, fragile skin can be reduced by use of a hydrogel. This is particularly relevant for neonate skin.

According to embodiments of the invention, respiratory rate algorithms are derived from chest wall movement that more accurately reflects true physiological function. Algorithms to derive SpO₂ with improved sensing accuracy in spite of motion artifact. Dual heart rate sensing coupled from both the ECG and high frequency accelerometer. Dual measurement systems to capture accurate respiratory rate. The measurement of respiratory rate derived through the ECG via impedance pneumonography is often times inaccurate and overestimates the true respiratory rate. Described herein is the ability to measure chest wall movement to derive respiratory rate along with traditional impedance pneumonography. Continuous cross-validation is possible. High frequency accelerometer enables electrode-free method that avoids the need for additional skin preparation for the measurement of heart rate and respiratory rate; signal processing enables distinction between chest wall movement associated with breathing compared to heart rate.

According to embodiments of the invention, the sensor network has advanced software function and interoperability enabling cloud storage, remote login, and secure communication. Further capabilities include the integration of third-party sensors, wearables, and other hardware systems as well as paired integration with electronic health records.

Medical applications of the sensor network include, but are not limited to, multimodal sensing, including pulseless electrical activity. The ECG on the chest can pick up perfect ECG, heart, etc., but the heart is not actually pumping blood (in instances of hypovolemic shock). The accelerometer or the PPG can corroborate or refute the presence of cardiac activity. Another aspect is assessing pediatric/neonatal pain: given that neonates are non-verbal, measurement and assessment of pain is difficult. Accordingly, the sensor network can provide various measures relevant to pain including, but not limited to heart rate, heart rate variability, respiratory rate, respiratory effort, crying time. This further enables the ability to notify a provider or practitioner where pain is assessed. Kangaroo mother care (KMC): For instance, during skin-to-skin or "kangaroo care" where a baby is placed on the mom's chest, the ECG sensors can be placed on the back or flank of the neonate. Algorithms enable the detection of baby position in space and classification of positions relevant to kangaroo care.

In certain embodiments, the pulse oximeter unit is placed on various locations of the body to derive a measure location specific pulse oximetry. Locations include but are not limited to: all four limbs, chest, back, abdomen, forehead. These locations enable assessment of local blood perfusion - for instance, in the context of the forehead, the SpO₂ can be used as a reflection of cerebral perfusion. Detection of apneic events: sudden decreases or cessation of respiratory rate followed by compensatory increases in heart rate and decreases in SpO₂ may suggest the need intervention; this intervention include notification of a healthcare provider or vibration of the sensor itself to trigger the neonate to change position or awake from sleep. Beyond critical care, these devices may be used in the post-surgical setting, the home setting, or in adult care settings.

In certain embodiments, the form factor includes a single chest unit sensor with all electronics and vital sign monitoring functionality.

In certain embodiments, the sensor network has the ability to create alarms based on preprogrammed or physician specified inputs. These alarms include both visual and audio notifications. These notifications can be displayed on an external display unit or onboard the sensor itself (either via an onboard LED or sound).

In certain embodiments, the optical unit of the photoplethysmograph is located on the infant's nail (toenail or fingernail). Adherence to the nail allows for an excellent device/skin interface. The nail is surface with negligible risk of injury and no risk of irritation.

In certain embodiments, an electromechanical motor is integrated within the sensor unit - gentle vibration is soothing for babies and often times induces sleep. The sensors can act as sensing-therapeutic couplers. The sensing of crying can trigger a gentle vibration that soothes the baby.

Certain aspects of the invention disclose a sensor network for wireless monitoring of physiological parameters comprising a plurality of time-synchronized sensor systems, wherein each sensor system comprises a sensor to monitor a physiological parameter; a bidirectional wireless communication system for wirelessly transmitting data to and from the plurality of time-synchronized sensor systems; and a remote reader in communication with the bidirectional wireless communication system for real-time display of the monitored physiological parameters, recording of the monitored physiological parameters, and/or alarm for an out of agreement state.

In certain embodiments, a first sensor system is configured to attach to a central patient region and a second sensor system is configured to attach to an extremity patient region.

In certain embodiments, the central patient region comprises a chest region, a neck region including the suprasternal notch area, or a head region including a forehead region or anterior fontanelle region of a neonate.

In certain embodiments, the extremity patient region comprises one or more of: a limb region, a foot region, a hand region, a toenail region or a fingernail region.

In certain embodiments, a sensor output triggers an indicator to indicate a vital sign is of lower confidence.

In certain embodiments, the sensor output alters an executable algorithm in a microprocessor of the sensor system.

In certain embodiments, the altered executable algorithm comprises one or more of a filter, a display parameter or a correction factor.

In certain embodiments, the sensor network further comprises a microprocessor for receiving the physiological parameter data from the plurality sensors; and an alarm module executable on the microprocessor to determine if at least two sensors are in an out of agreement condition.

In certain embodiments, the sensors monitor a plurality of physiological parameters to provide multi-modal monitoring of a critical parameter to reduce false positives and notify a caregiver of a sensor aberrant signal capture condition.

In certain embodiments, the sensor network further comprises at least three independent sensors that monitor the same physiological parameter, wherein the sensors measure a different physical parameter, and the different physical parameters each provide an independent measure of a critical vital sign, and are optionally located at different locations on a patient body.

In certain embodiments, the sensor network further comprises a processer that executes an agreement calculator module to send a signal to an alarm to notify a clinician of an out of range vital sign for a condition corresponding to at least three independent sensors in agreement of the out of range vital sign; or send an aberrant sensor signal condition for an out of range vital sign measured by only one of the at least three sensors; thereby increasing sensor network sensitivity, positive predictive value and reducing false negatives.

In certain embodiments, the sensor network further comprises an accelerometer sensor for use with a motion artifact module to identify a vital sign as subject to motion artifact and/or to correct of motion artifact.

In certain embodiments, the critical parameter is one or more of heart rate, brain activity, temperature, patient motion, respiration or blood-flow.

In certain embodiments, the sensors are one or more of, an ECG sensor, and EKG sensor, a pulse oximeter sensor, a temperature sensor, a blood pressure sensor, an accelerometer, or an acoustic sensor; and a microprocessor constantly compares values from each of the sensors and an alarm module generates a signal for the out of agreement condition between the at least two sensors.

In certain embodiments, the sensor network further comprises an actuator in wireless communication with the remote reader configured to receive an actuation signal to actuate underlying tissue.

In certain embodiments, the actuator is one or more of an electromechanical motor, a heater, and an electrical stimulator.

In certain embodiments, the reader provides continuous display of a critical parameter even under a sensor-fail condition.

In certain embodiments, the sensor network further comprises a wireless power unit for wirelessly powering the sensors.

In certain embodiments, the sensors operate continuously, including over a time period that is greater than 8 hours, or between about 8 hours and 1 day, without recharging.

In certain embodiments, the sensors measure one or more physiological parameters that are crying time, crying frequency, swallow count, swallow frequency, chest wall displacement, heart sounds, core body position, skin temperature differentials, asynchronous limb motion, speaking.

In certain embodiments, the sensor network is configured for use with a pediatric or neonate patient.

In certain embodiments, the monitored vital signs provide an indication of pain magnitude.

In certain embodiments, the sensor network further comprises an actuator to generate a force configured to provide a stimulus to a patient.

In certain embodiments, the patient is a neonate or a pediatric patient, and the actuator comprises an electromechanical motor configured to provide a soothing vibratory force to the patient.

In certain embodiments, the sensor detects crying and provides a signal to automatically actuate the actuator and sooth the patient.

In certain embodiments, the sensor network further comprises a remote controller configured to have two-way wireless communication with the sensors.

In certain embodiments, the remote controller is operably connected to a patient database.

In certain embodiments, the remote controller is a hand-held device or a portable device having a graphical user interface to display the plurality of monitored physiological parameters.

In certain embodiments, the remote controller and the receiver are integrated as a single unit.

In certain embodiments, the sensor network is capable of operating wirelessly for a period of at least 24 hours without a source of external power.

In certain embodiments, the time synchronized sensors synchronizes the physiological parameter data from the plurality sensors independent of sensor position, including for multiple spatially separated sensors used to determine a common physiological parameter.

In certain embodiments, each of the plurality of sensor systems comprise: a plurality of electronic components; a serpentine interconnect that electrically interconnects different electronic components; an elastomeric encapsulation layer that surrounds the plurality of electronic components and serpentine interconnect to form a bottom tissue-facing surface and a top environment-facing surface; an antenna for wirelessly communicating a physiological parameter measured from the sensor system; wherein the bottom tissue-facing surface is configured to conform to a skin surface.

Certain aspects of the invention also disclose a method of monitoring one or more vital signs of a neonate or a pediatric patient, the method comprising the steps of: providing the sensor network disclosed above; conformally contacting the first sensor with a first region of the patient; conformally contacting the second sensor with a second region of the patient that is spatially separated from the first region; detecting with the first and second sensors one or more physiological parameters that are related to a vital sign; and wirelessly transmitting the detected physiological parameters or the vital sign to a remote controller unit; thereby monitoring one or more vital signs.

In certain embodiments, the vital sign is one or more of: heart parameter (rate, intensity, variability), respiratory parameter (rate, intensity, variability), temperature, oxygenation (level, variability, minimum, maximum), vocalization parameter (crying duration, frequency, intensity), swallow parameter, and blood pressure.

In certain embodiments, the method further comprises he step of associating the wirelessly transmitted the physiological parameters with a unique patient identifier; thereby identifying the physiological parameters with a patient.

In certain embodiments, the method further comprises the step displaying the transmitted physiological parameters in a display having a graphical user interface.

In certain embodiments, the method further comprises the step of securing the display of transmitted physiological parameters by requiring a secure login step before a user can access the transmitted physiological parameters.

In certain embodiments, the user is a healthcare professional and a patient or family member, further comprising the step of wirelessly communicating between the healthcare professional and the patient or family member based on the detected physiological parameters or the vital sign.

In certain embodiments, the method further comprises the step of the user that is a remote healthcare professional securely sending a command to a proximate healthcare professional in proximity to the patient based on the transmitted physiological parameters.

In certain embodiments, the method further comprises the steps of determining a vital sign from at least two different sensors within two different sensor systems; comparing the at least two vital sign determinations; identifying from the comparing step one of a normal vital sign reading condition; an aberrant sensor output condition; or an alarming vital sign reading condition.

In certain embodiments, the aberrant sensor output condition is one or more of a lead off state, wherein a bottom surface of a sensor system is not in intimate contact with an underlying patient surface; a patient motion artifact; or an output discrepancy relative to at least two other sensors.

In certain embodiments, the aberrant sensor output condition is identified for at least one vital sign from a first sensor that differs from another vital sign from a second sensor that is at least 25% different.

In certain embodiments, the method further comprises the step of identifying the sensor having the aberrant sensor output condition and alerting a caregiver of the condition to take action to correct the sensor output.

In certain embodiments, for an alarming vital sign reading condition, the method further comprises generating an alarm signal to alert a health care professional.

In certain embodiments, the vital sign is one or more of temperature, heart rate, respiratory rate or movement.

In certain embodiments, the physiological parameter is position, and the vital sign corresponds to a baby position that is a laying position.

In certain embodiments, the wireless transmitting is by a Bluetooth protocol.

In certain embodiments, the vital sign is respiratory rate derived from a sensor that measures chest wall movement.

In certain embodiments, the vital sign is SpO₂, the method further comprising the step of improving SpO₂ accuracy by accommodating any motion artifact.

In certain embodiments, the vital sign is heart rate, and the method further comprises detecting heart rate from both an ECG sensor and a high frequency accelerometer, thereby providing continuous cross-validation.

In certain embodiments, the vital sign is respiratory rate, and the method further comprises measuring a chest wall movement with a first sensor and impedance pneumonography with a second sensor, thereby providing continuous cross-validation.

In certain embodiments, the method further comprises the step of storing the transmitted physiological parameters or vital sign in a network of servers.

Certain aspects of the invention further disclose a battery-powered, wireless (e.g., Bluetooth 5 enabled) vital signs monitoring system that exploits a bi-nodal pair of thin, low-modulus measurement modules, capable of gently and non-invasively interfacing onto the skin of neonates, even at gestational ages that approach the limit of viability. The key distinguishing features of this technology includes low-battery power operation enabling at least 24-hour continuous use between charges while enabling monitoring of a full suite of vital signs. The designs enable measurement of traditional vital signs in addition to advanced physiological parameters not currently measured. The skin interface and electrical/mechanical design of the sensor allows for safe integration with fragile neonatal skin even during life-saving interventions such as cardiac defibrillation.

Also disclosed herein are methods of monitoring using any of the sensor networks, sensor systems and electronic components described herein. Also provided herein are sensor networks for carrying out any of the methods described herein.

These and other aspects of the present invention are further described below. Without intent to limit the scope of the invention, examples according to the embodiments of the present invention are given below. Note that titles or subtitles may be used in the examples for convenience of a reader, which in no way should limit the scope of the invention. Moreover, certain theories are proposed and disclosed herein; however, in no way they, whether they are right or wrong, should limit the scope of the invention so long as the invention is practiced according to the invention without regard for any particular theory or scheme of action.

### SENSOR NETWORK OVERVIEW AND ELECTRICAL COMPONENTS

Each of the sensor systems comprises a plurality of electrical components, as schematically illustrated in FIGS. 2A-2G, depending on the functionality of each sensor system. Each of FIGS. 2A-2C and 2F shows a schematic of an electrocardiography (ECG) sensor system according to different embodiment of the invention, which is configured to measure additional physiological parameters, such as temperature and position/motion. The electrodes and accelerometer are connected to complementary electrical components such as amplifiers and filters. Each of FIGS. 2A, 2B, 2D and 2G illustrates a photoplethysmography (PPG) sensor system according to different embodiment of the invention, which includes a temperature sensor. The PPG sensor includes a photodetector and optical source, such as an infrared or red light, including a light emitting diode (LED). Associated electronic components include an amplifiers and filters. Wireless communication to a microprocessor, including Bluetooth low energy (BLE) communication.

Representative geometries are provided in FIG. 4 for appendage mounting, including, as illustrated, a foot. Details of two sensor systems are summarized in FIG. 3. The combination of time-synchronized sensor systems, including such as those of FIG. 3, provides a number of important benefits. For example, multiple vital signs can be independently monitored and cross-checked against one another to provide higher-fidelity monitoring and information to distinguish between serious medical events compared to sensor malfunction arising, for example, from bad contact with the skin. The sensor systems have functional characteristics of being wireless, with a long battery life, and having, at most, a biologically-gentle adhesive such as a hydrogel, between the sensor system and the skin. The chest sensor system can measure ECG, heart rate, temperature (medical grade), respiratory rate (RR) and motion detection. The foot sensor system can measure oxygen saturation (SpO₂) and temperature. Together, the systems can measure blood pressure from pulse arrival time (PAT) or pulse transit time (PTT).

FIGS. 2C-2D is a schematic illustration of one sensor system, with the top panels illustrating the undeformed sensor system, a stretched sensor system, and a folded or twisted sensor system. The bottom panel is a schematic illustration of the various positions of the electrical components and the ability of the system to accommodate various applied forces, including stretching (buckling) and folding (bending) by use of thin layered electronics, serpentine electrical interconnects, and positioning of stress-sensitive components (e.g., electrodes, integrated circuits and chips, power source, communication components, and processers). The system may be encapsulated with an encapsulation layer, including a continuous encapsulation layer as there is no need for external wire connections.

A similar sensor system, but configured for appendage or limb mounting, is illustrated in FIG. 2E. The upper panels illustrate the sensor configured to be wrapped around an appendage or limb.

The PCB can be fabricated using 3/3 mil trace spacing, down to 3/2 mil trace spacing depending on the application of interest. The PCB may comprise a plurality of stacked layers, with a thickness of about 6.92 mil (e.g., less than about 0.2 mm).

The sensing capabilities of the sensor network and systems provided herein provides a range of sensing capabilities, including measuring parameters related to one or more of heart rate, heart rate variability, respiratory rate, skin temperature, pulse oximetry, ballistocardiography, respiratory effort, crying time, swallowing count, cuff-less blood pressure.

Charging platform preferably has a receiver dimension less than 15 × 15 mm², a power transfer capacity up to 200 mW, and an effective wireless charging distance up to about 2 to 3 cm. The wireless charging is similar to the standard WPC Qi, although the actual Qi standard tends to be inadequate for the instant systems. The receiver-side requirement is a coil with resonance frequency of 110 - 205 kHz, where a bigger coil for resonant frequency is provided herein. An additional magnetic flux shield is necessary for higher efficiency and inductance-coil turn ratio, but such a shield adds unnecessary bulkiness to the sensor systems, making it difficult to achieve long-term wearability.

The NFC and Qi standards are compared, including by frequency range (13.56 MHz vs. 110-205 kHx), charging power range (100-500 mW vs. 5-120W) and typical receiver size (> 10 × 10 mm² vs. 45 × 45 mm²).

An exemplary electronic component is an NFC Reader IC, such as ST25R3911A NFC Reader IC (FIG. 1G). An NFC initiator/HG reader can be used for the charger platform. Up to 1.4W of transmitting power output with differential driving support is obtained. In this manner, transmitter control and wake-up mode for power efficiency is achieved. Small size and weight sensor systems are achieved, that are conformable to a body surface and light-weight, such as 4.7cm × 2.2 cm × 0.5 cm (4.8 g) or 4.0 cm × 1.9 cm × 0.5 cm (4.6 g).

### SOFTWARE AND ALGORITHMS

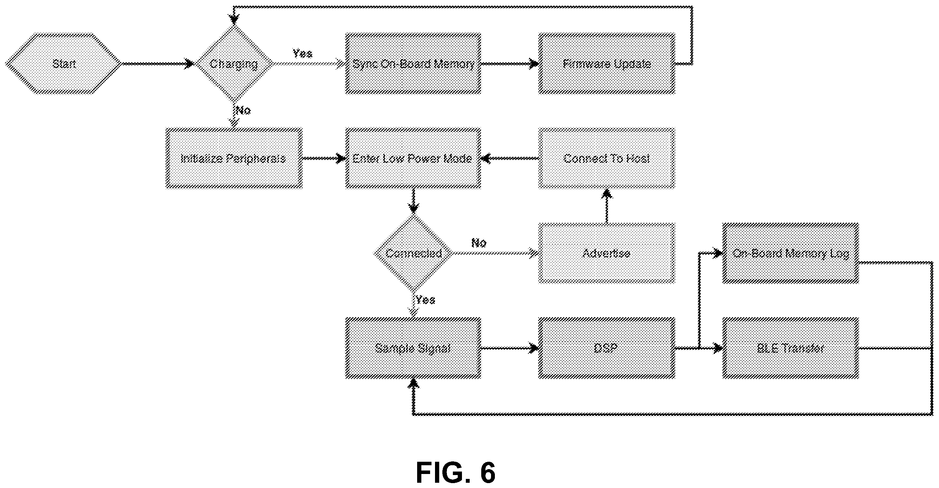

FIG. 6 illustrates a flowchart of operation of a sensor network system according to one embodiment of the invention. In the exemplary embodiment, the operation of the sensor network starts to check whether the battery module needs to be charged or not. If the charging is needed, the system synchronizes on-board memory and perform firmware update and then go back to check if the battery module needs to be charged. If no charging is needed, the system initializes peripherals and then enters a low power mode, followed by checking whether the sensor systems are connected or not. If no, the sensor network system advertises to request to connect to a host, and then enters the low power mode. If yes, the sensor systems sample signals and the sampled signals are then processed with digital signal process (DSP). The processed signals are subjected to BLE transfer and meanwhile writes the transfer in an on-board memory log. Then repeating the signal sampling process. The systems are compatible with any number of hardware devices and platforms, including mobile phones, handhelds and computers.

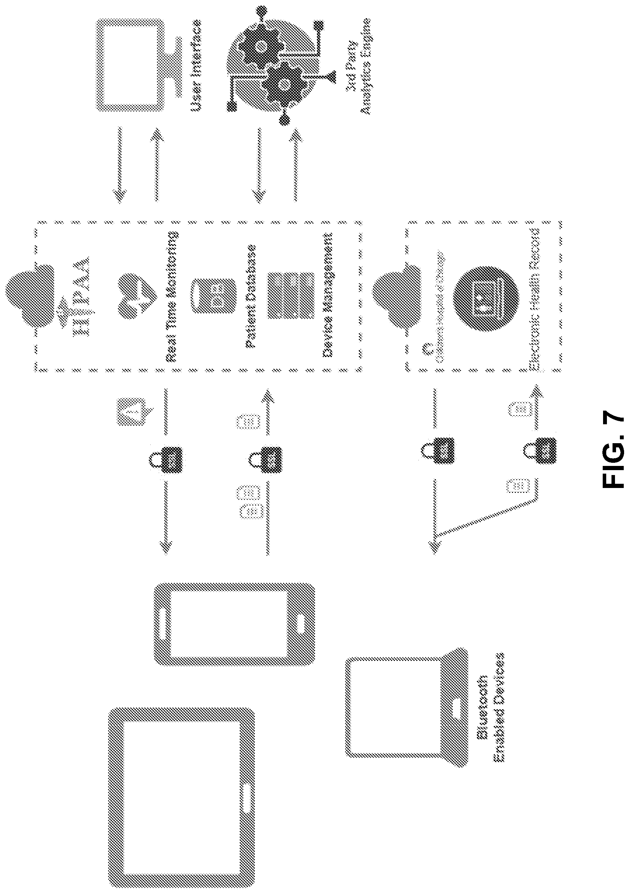

Referring to FIG. 7, a high-level cloud architecture schematic is shown according to one embodiment of the invention. Generally, the sensor network for real-time monitoring, patient database storing data of real-time monitoring from the sensor network, and device management are operably in bidirectional wireless communication with user interface and/or third party analysis engine, and also operably in wireless communication with mobile devices, such smartphones and tablets. In addition, the monitor data may be stored in hospital's electronic health record system that may be in communication with a Bluetooth enabled device.

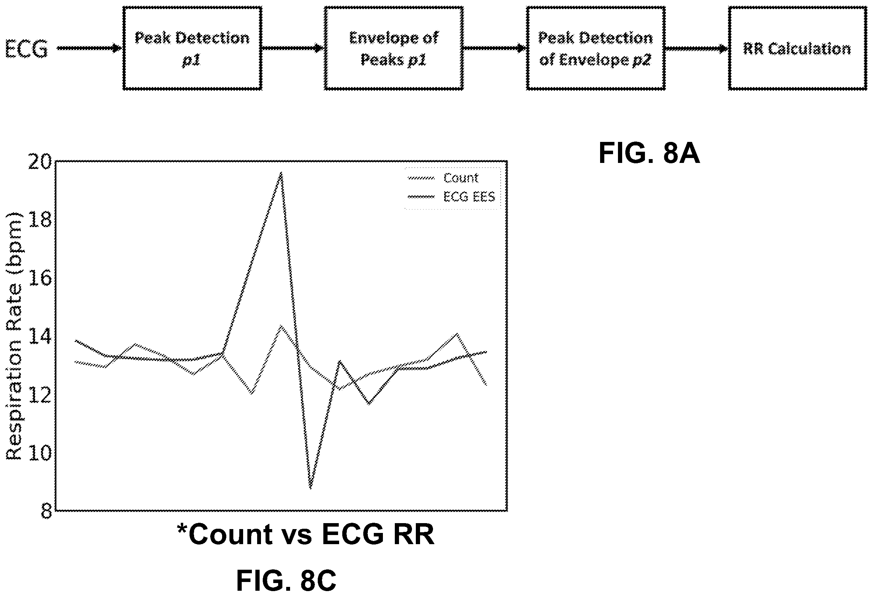

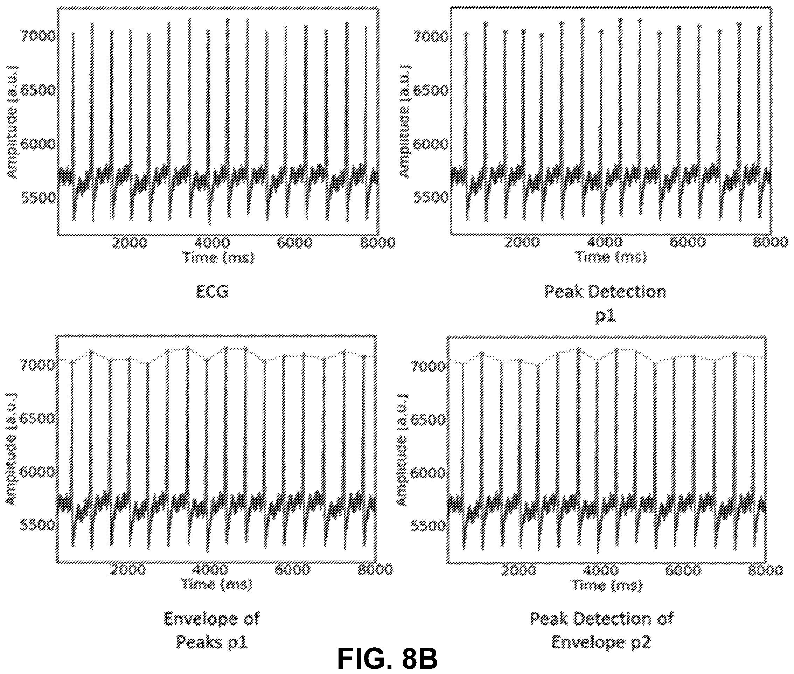

FIG. 8A shows an algorithm using an ECG sensor for respiratory rate (RR) estimation according to one embodiment of the invention. FIG. 8B illustrates the RR estimation using the algorithm shown in FIG. 8A, based on the ECG signal measured by the ECG sensor, with a plot of the actual count compared to the ECG EES provided in FIG. 8C. For example, as shown in FIG. 8B, the peaks p1 (top right panel) is detected from the measured ECG (top left panel), then the evelope of the peaks p 1 is determined (bottom left panel). The peaks p2 of the determined peak envelope is detected (bottom right panel) for the RR estimation.

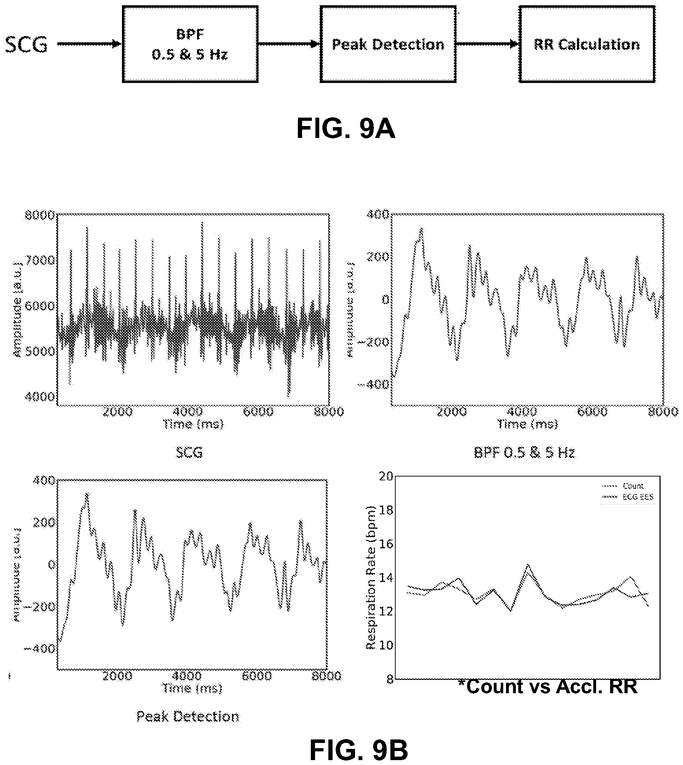

FIG. 9A shows an algorithm using chest wall movements for the RR estimation according to another embodiment of the invention. FIG. 9B illustrates determination of the RR using the algorithm shown in FIG. 9A, based on the measured chest wall movements, for example, measured by an accelerometer. In this embodiment, the SCG signal associated with the chest wall movements (top left panel) is processed with a bass pass filter (BPF) to obtain a band pass filtered (f*_{c1}* = 0.5 Hz, f*_{c2}* = 5 Hz) signal (top right panel), whose peaks is then detected (bottom left panel) for the RR estimation.

In these exemplary embodiments, the sensor network is characterized as multi-modal, in that different sensors may be used to determine a same physiological parameter. This multi-modal input provides the ability to achieve much more reliable data readouts and intervention, which is important in critical care applications, and neonatal care.

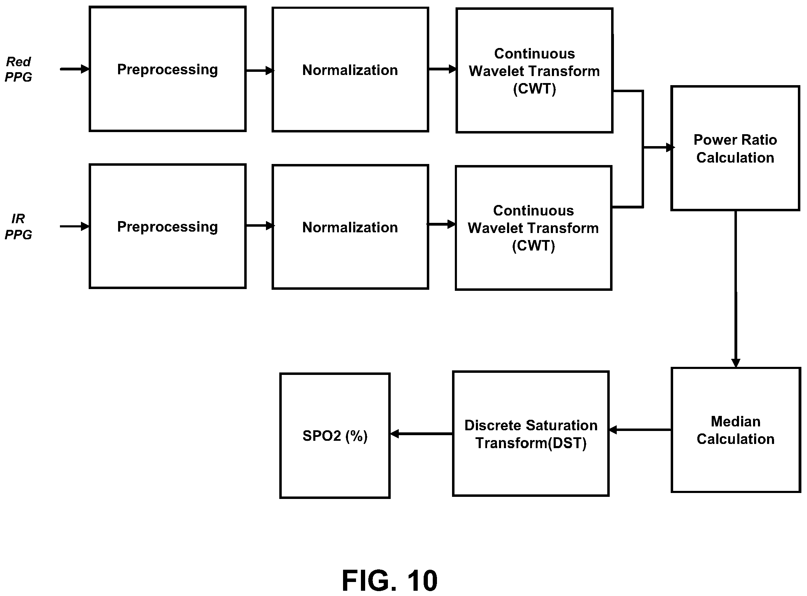

In certain embodiments, pulse oximetry (SpO₂) can be determined by continuous wavelet transform (CWT) and discrete saturation transform (DST). FIG. 10 summarizes an algorithm of the CWT and DST for the pulse oximetry. In the exemplary embodiment, calculation of SpO₂ involves with algorithms known for an effective motion artifact reduction, which is critical to calculate accurate value as babies in NICU and PICU are often fidgeting. Band pass filtered (f*_{c1}* = 0.5 Hz, f*_{c2}* = 5 Hz) and normalized PPG signals are processed further with the CWT that constructs continuous time-frequency analysis of the signals. The CWT is effective in detecting rapid changes in frequency in the time domain that can be caused by motion-driven artifact, which serves as the first motion artifact reduction stage. Following with the power ratio calculation and getting the median value, the signal processing further suppresses motion artifact by the DST algorithm. The DST algorithm involves with an adaptive filtering and determination of family of the reference noise signal and true signal based on optical density ratio. The adaptive filtering automatically cancels noise content, which is based off the pre-determined reference. This series of signal processing is occurring in real-time to yield an accurate SpO₂ value.

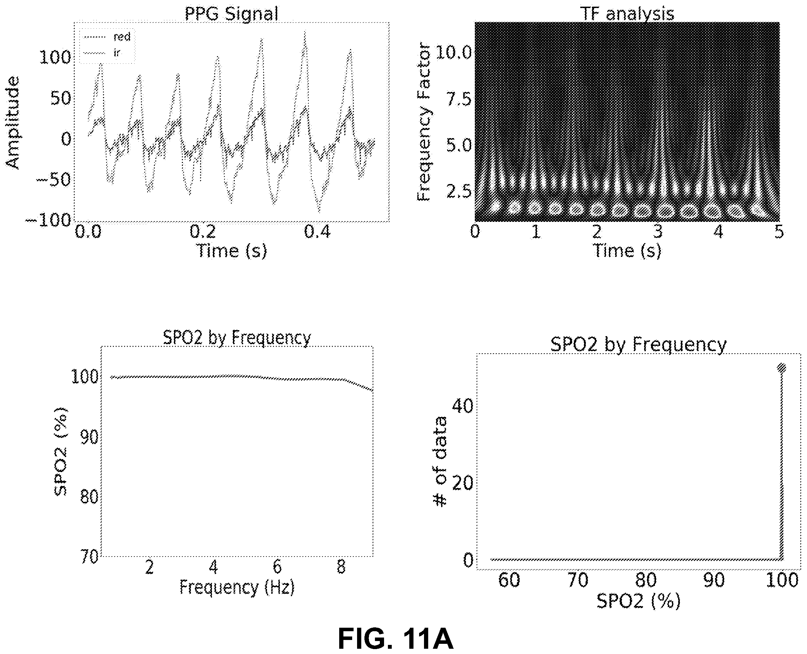

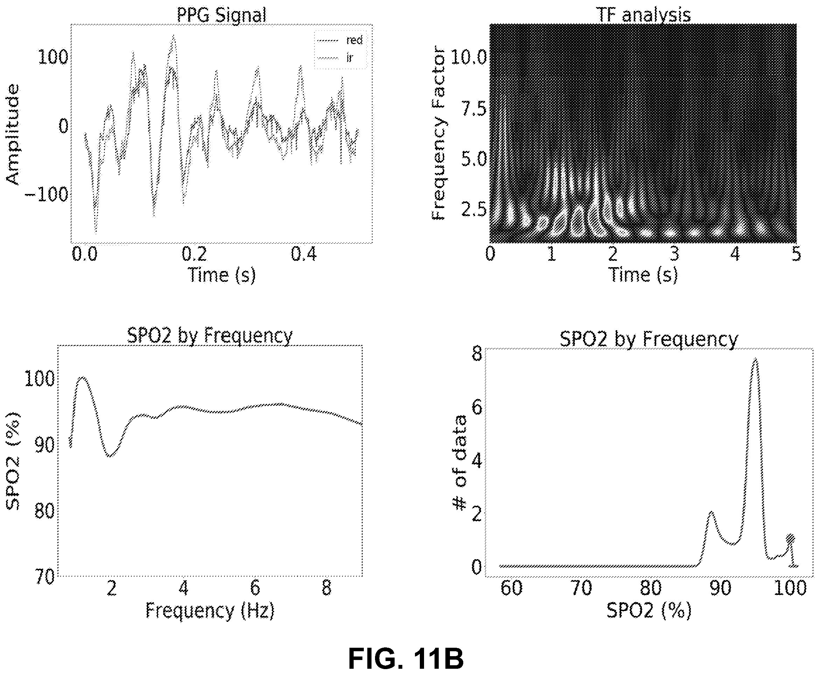

FIGS. 11A-11B illustrate resultant outcomes of SpO₂ for a clean signal (without motion artifact) and a noisy signal (motion contaminated signal), respectively, where a motion-resistant algorithm is used, which is based on the CWT. A time-frequency domain (TF) analysis distinguishes real pulsatile response and motion induced response.

### SENSOR PERFORMANCE

Referring to FIG. 12, for heart rate, the mean difference from the experimental system compared to standard-of-care system was -0.47 beats per minute aggregated across 18 subjects, according to certain embodiments of the invention. The standard deviation of the mean difference was 3.0 beats per minute. Thus, 95% of the data fall within a discrepancy less than 6.0 beats per minute. The agreement of the experimental wireless system remained comparable at lower heart rates (<100 beats per minute) and higher heart rates (>170 beats per minute).

Referring to FIG. 13, for blood oxygenation, the mean difference was 0.11% and the standard deviation of the mean difference was 1.8% between the experimental system and the standard-of-care system aggregated across 16 subjects, according to certain embodiments of the invention. Thus, 95% of the data fall within a discrepancy less than 3.6% blood oxygenation. The agreement of the experimental wireless system remained comparable at both lower blood oxygenation (<90%) as well as higher blood oxygenation (>96%). Two subjects had intravenous lines or other medically necessary equipment on both lower extremities and excluded.

Referring to FIG. 14, for respiratory rate, the mean differences was -2.0 breaths per minute with a standard deviation of mean differences of 8.7 breaths per minute between the experimental system and the reference system according to certain embodiments of the invention. The higher variation for respiratory rate may relate to differences in the measuring modality of respiratory rate. Respiratory rate derived by the experimental system is through the ECG while the reference Phillips monitor utilizes thoracic impedance pneumography). Similar performance is noted for measurements across low and high respiration rates (<30 and >70 breaths per minute).

FIG. 15 shows the heart rate determined by a sensor network according to certain embodiments of the invention, illustrating the equivalent performance to an FDA-approved device.

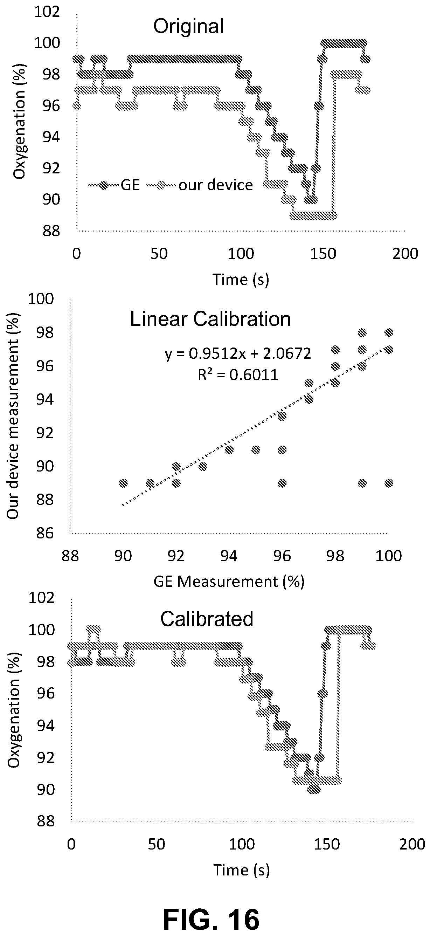

FIG. 16 illustrates good agreement between an ECG sensor system according to one embodiment of the invention and an FDA-approved gold standard device. The top panel shows original data measured by both the ECG sensor system and the FDA-approved gold standard device. The middle panel shows a linear calibration of the data measured by the ECG sensor system and the FDA-approved gold standard device. After the calibration, as shown in the bottom panel of FIG. 16, the measurements between the two different systems are well-correlated, with a difference after calibration of 1.1±1.9. Similar equivalence is illustrated for SpO₂ (%) and temperature. Such calibration factors may be used in any of the algorithms, to further improve sensor accuracy.

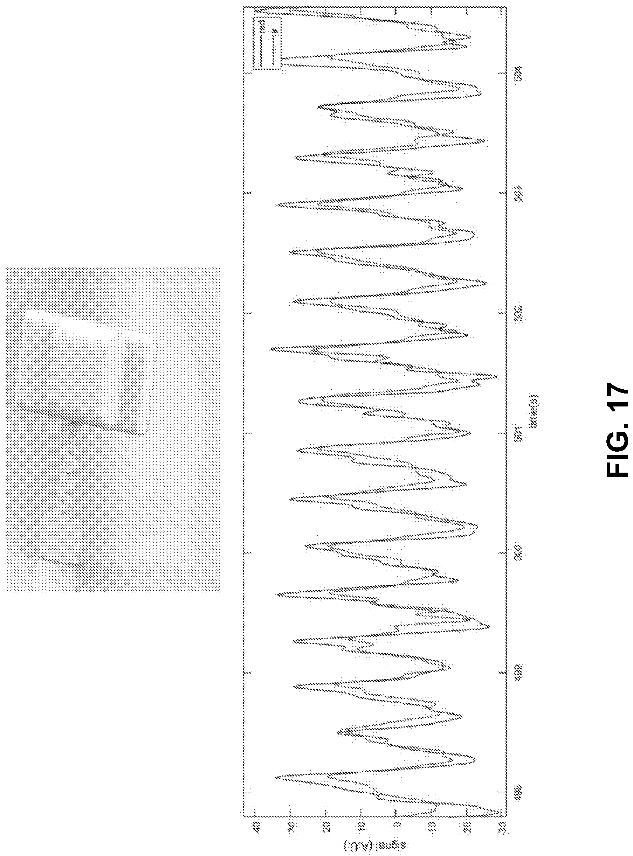

FIG. 17 illustrates a fully wireless sensor platform (top panel) according to one embodiment of the invention and a neonatal forehead measurement (bottom panel) using an IR wavelength of 860 nm, red wavelength of 660 nm, source detector distance of 3.7 mm measured by the wireless sensor platform.

### SKIN SAFETY

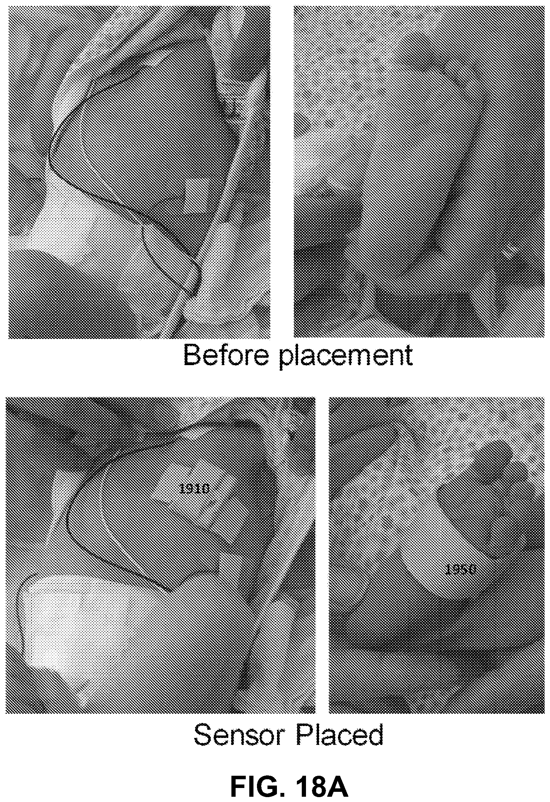

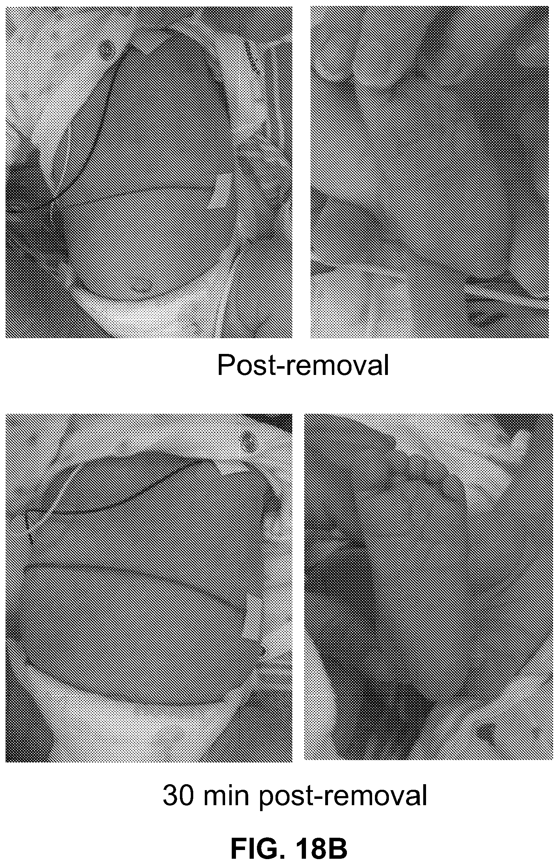

The sensor network is deployed on two premature and low birth weight neonates (<2000 g). The first sensor system 1910 is deployed in the chest adjacent to existing monitoring electrodes. The second sensor system 1950 is placed on the foot for pulse oximetry. The standard-of-care pulse oximeter is placed on the contralateral limb. The first sensor system is placed on the central chest. The skin underlying the first and second sensor system 1910 and 1950 shows no visible signs of erythema, irritation, blistering, or erosions after 15 minutes of sensor removal, which is illustrated in FIGS. 18A-18B.

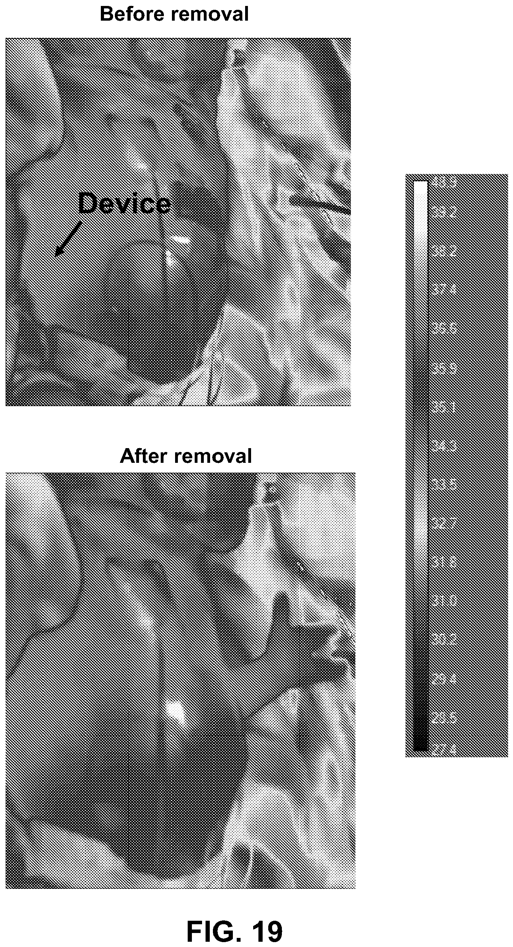

In certain embodiment, the sensor systems are used to evaluate thermal load provided to the underlying skin. Skin temperature after 24 hours of a chest-mounted sensor found the device at 35.4°C and the skin temperature after removal at 36.9°. This means there is not any substantial heat generation or transfer to the underlying tissue, as shown in FIG. 19. Similar results are obtained for an appendage sensors mounted to the foot. Analysis of thermal images of the device and skin during prolonged use and after removal reveals the sensor systems do not generate detectable heat.

### TEMPERATURE

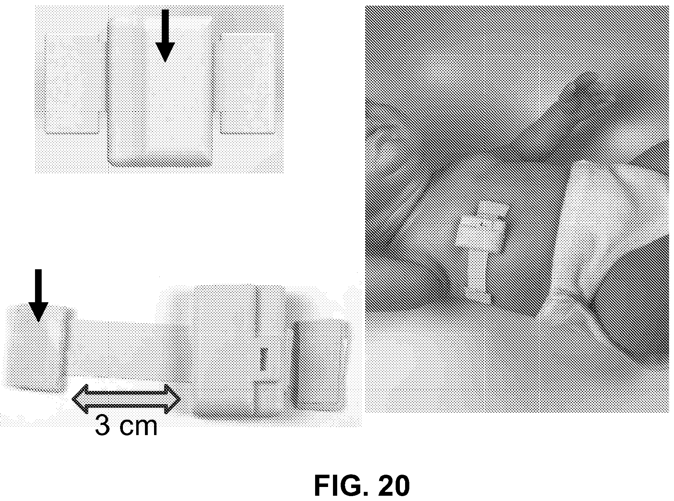

The left panels of FIG. 20 illustrate two different configurations for a temperature sensor, as indicated by the arrow. In the top-left panel, the temperature probe is located with the auxiliary electrical components. In the bottom left panel, the temperature probe is moved a distance of about 3 cm from the other electrical components. The image on the right illustrates mounting of the device to the skin. The purpose is to further improve the correlation between device and core temperature measurement.

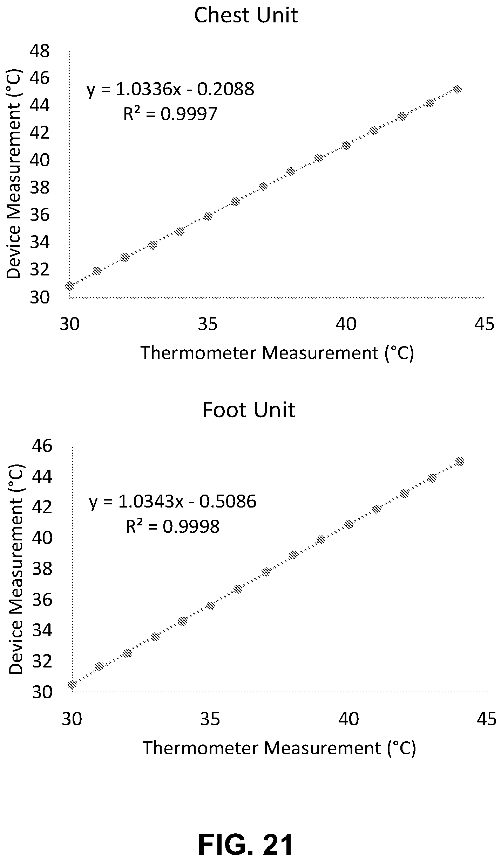

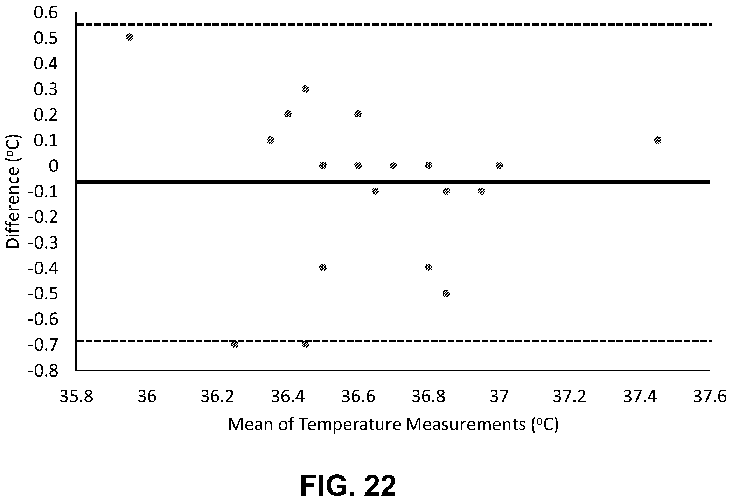

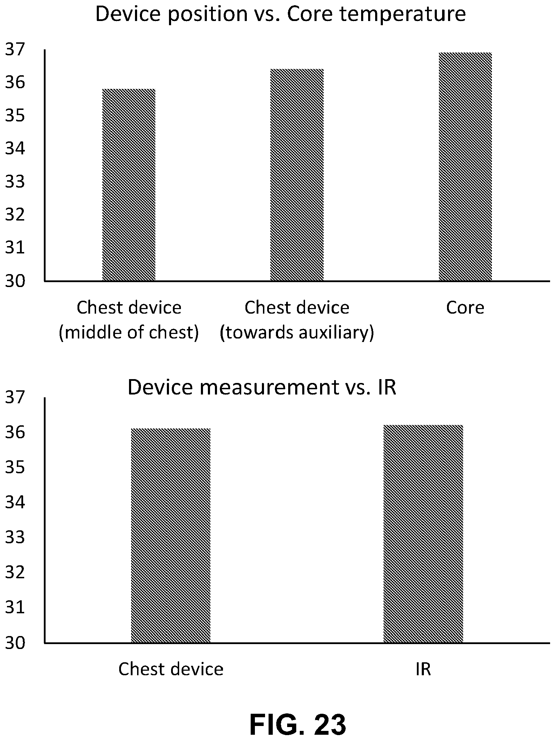

FIG. 21 illustrates a temperature sensor calibration for a chest unit (top panel) and a foot unit (bottom panel). A medical grade temperature sensor is calibrated with a thermometer, resulting in temperature precision of the chest unit and the foot unit of ± 0.09°C and ±0.08°C, respectively. FIG. 22 illustrates correlation of temperature measurements showing the instant temperature sensors in good agreement with a gold-standard FDA-approved temperature sensors, where the mean difference is 0.07°C. Temperature measurement results using the different devices of FIG. 22 are provided in FIG. 23 (top panel) compared to core temperature. The bottom panel of FIG. 23 illustrates good device agreement with infrared measurement.

### KANGAROO MOTHER CARE (KMC)

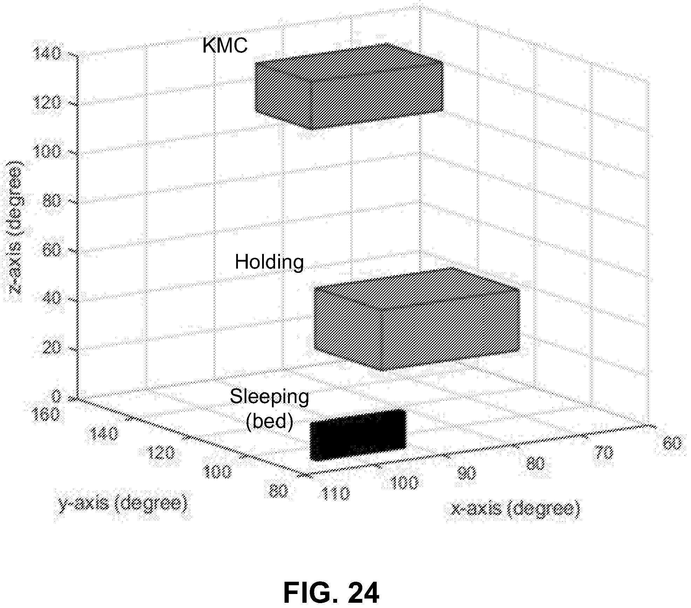

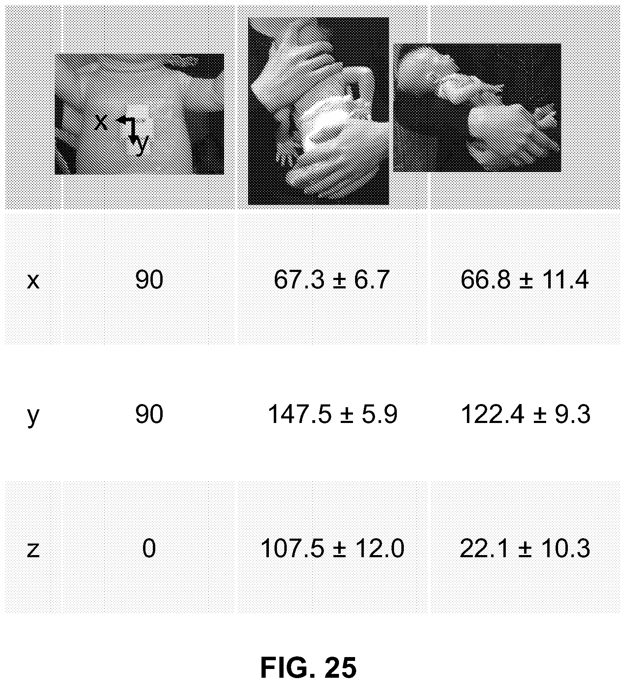

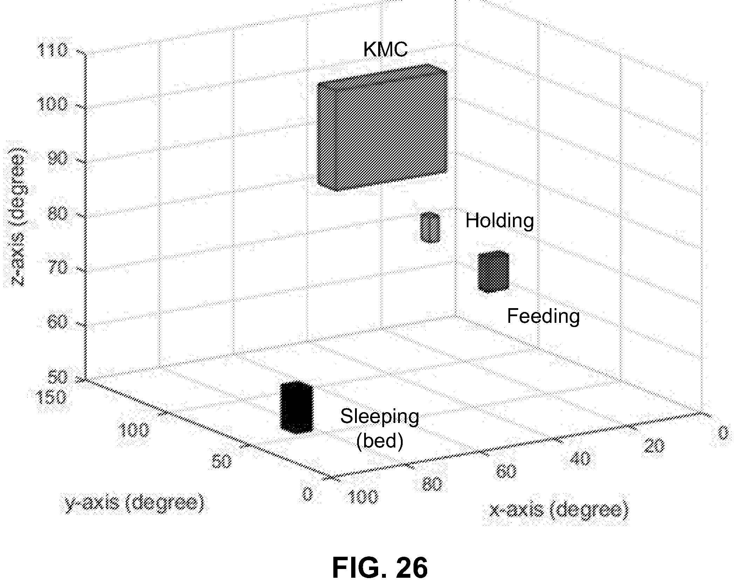

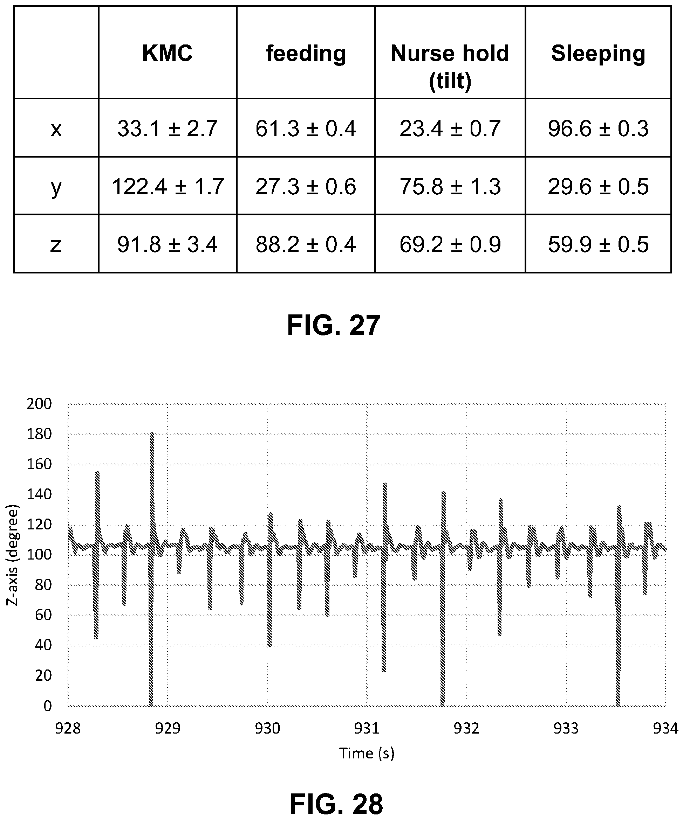

In certain embodiments, KMC can be simulated, where the neonate is placed on the mother's chest, held, or sleeping on a bed, with position monitored by a sensor network having an accelerometer in a sensor system, as shown in FIGS. 24-27. FIG. 24 is a graphical representation of three-dimensional sensor position (x, y, z) for a sleeping, held in arms and held against body in a KMC position. FIG. 25 shows the average (x, y, z) locations determined by a sensor system having an accelerometer, for an infant in different positions reflected by the photographs. FIG. 26 is a graphical representation of the location results for a sleeping, feeding, holding and KMC position. FIG. 27 is a summary of the measure (x, y, z) positions according to certain embodiments of the invention. Furthermore, an accelerometer sensor can be used to provide a visual reproduction of the baby position with time, and associating body position with one or more monitored physiological parameters.

Burping of a neonate in a clinical study is summarized in FIG. 28. The information can be compared to a video recording as an in-lab reference. Previously observed peaks are regenerated, and are well correlated with a 4 Hz frequency.

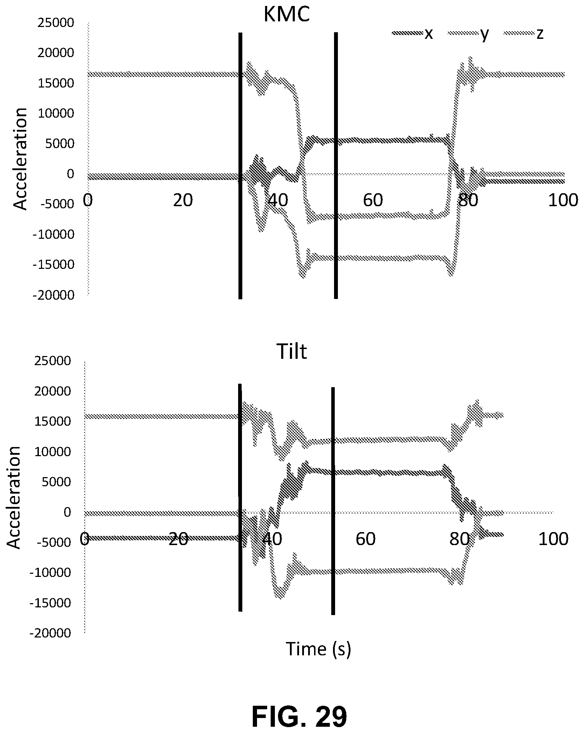

FIG. 29 summarizes a plot of in-lab simulation showing x, y, z postion, as a function of time.

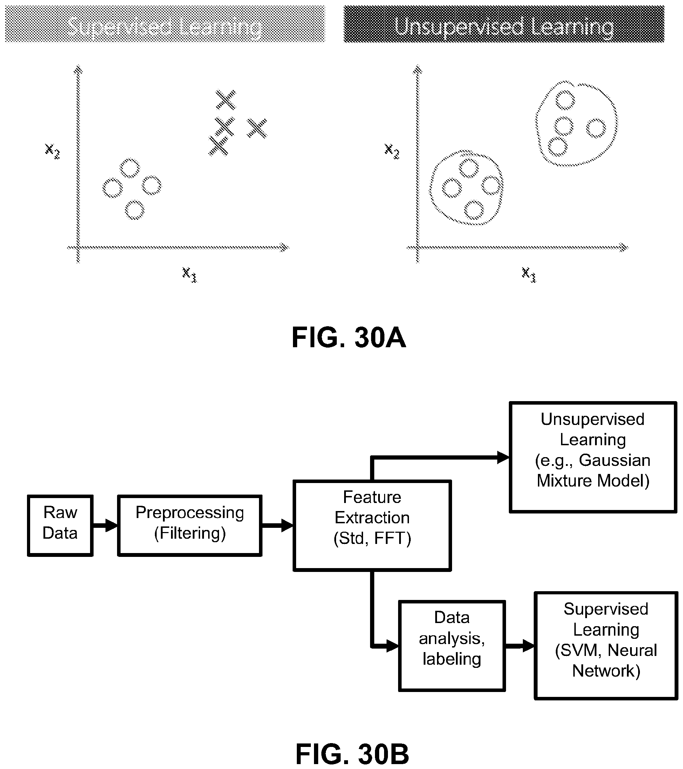

A variety of algorithms is useful for classifying sensor data, including sensor positions, as summarized in FIG. 30A. For example, various supervised learning (SVM, neural network) and unsupervised learning (e.g., Gaussian mixture model) may facilitate classification of sensor positions. FIG. 30B shows a flowchart of an algorithm for classifying the sensor data according to one embodiment of the invention. Generally, the algorithm for classifying the sensor data starts with preprocessing (filtering) raw data. Then the feature extraction is performed on the preprocessed data, using fast Fourier transform (FFT) and/or standard deviation (STD). The extracted features can be clustered by unsupervised learning (e.g., Gaussian mixture model) or by data labeling and then supervised learning (SVM, neural network).

For example, for a support vector machine, a supervised learning algorithm may be implemented. This involves finding a hyperplane with biggest margin between its offsets that would "cut" classes of data. The characteristics of SLM is that they are relatively simple to implement, effective in binary classification, and can perform both linear and non-linear classification. In one embodiment, real-time classification can be achieved, such as related to posture (lie, stand), tilt (KMC, tilted) and movement (still, move). A real-time plot of these parameters can be obtained.

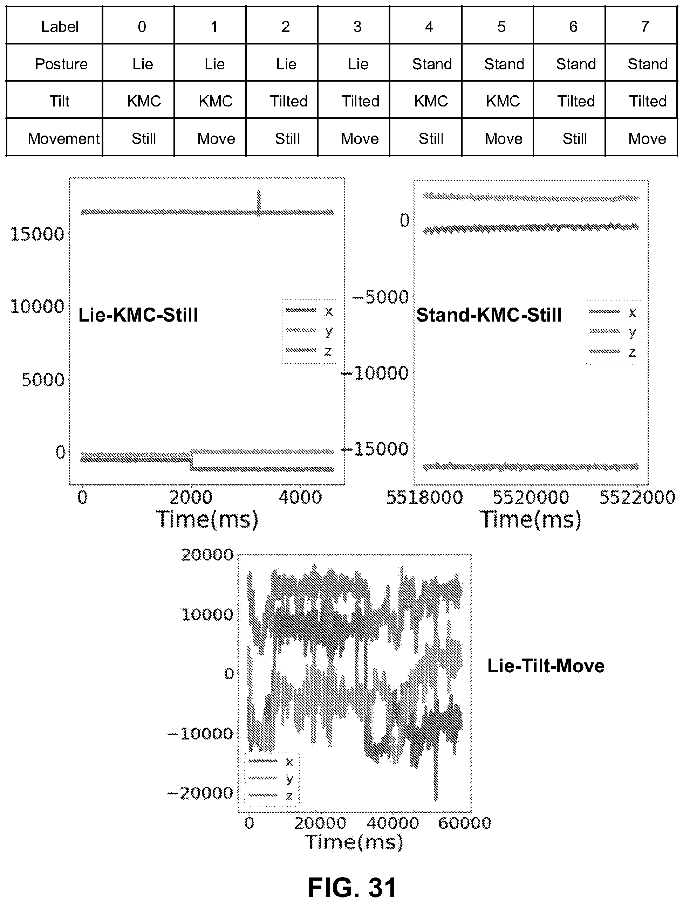

FIG. 31 illustrates data labeling (top panel) for posture, tilt and movement (total of 8 labels), based on an accelerometer sensor system, and data extracted from KMC 24-hour data measurements (middle and bottom panels) according to certain embodiments of the invention.

### USER INTERFACE

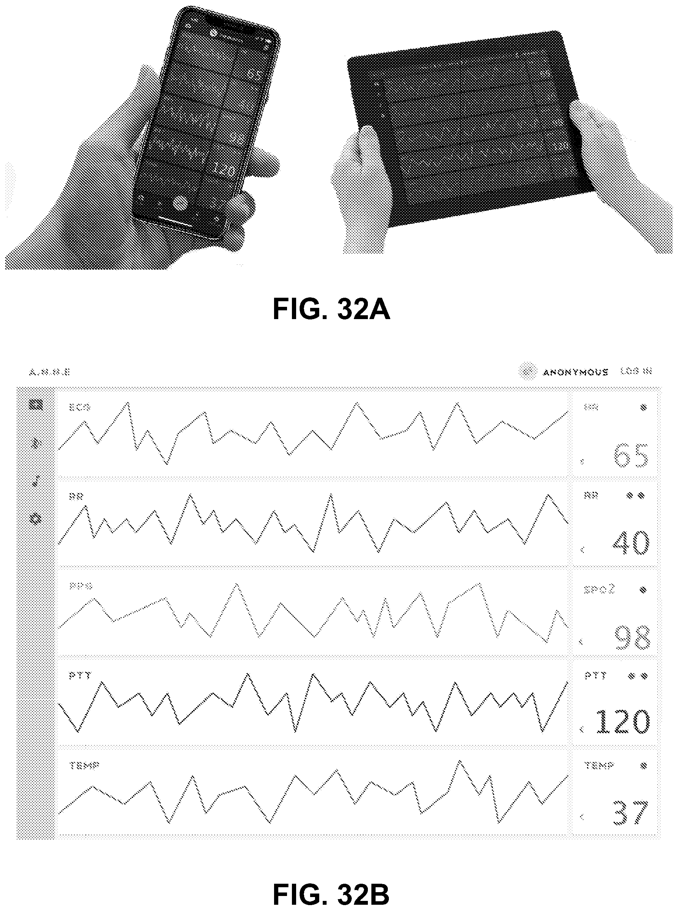

According to the invention, the sensor network is highly configurable and user-friendly, particularly in terms of a user interface that rapidly and naturally conveys a range of information. Various examples of the user interface are provided in FIGS. 32A-38.

As shown in FIG. 32A, the remote reader may be a handheld device such as a smartphone, tablet or the like. Of course, the wireless transmission makes the sensor network compatible with any number of reader platforms. The reader may display real-time or stored data. FIG. 32B illustrates displayed parameters related to ECG, RR, SpO₂, PTT and temperature, including a running average, instantaneous reading, and time-plotted.

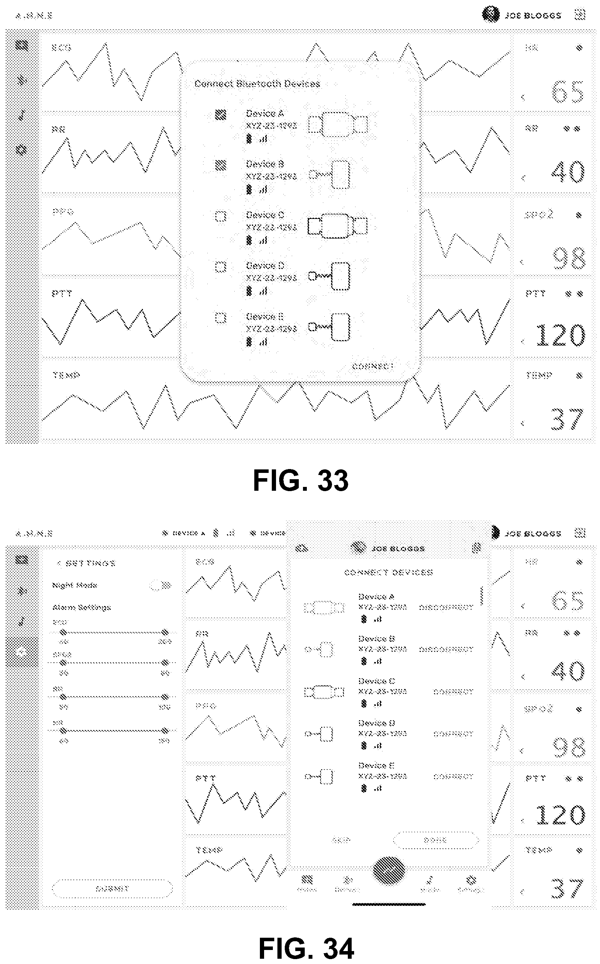

According to certain embodiments as shown in FIG. 33, the reader can connect to various sensors, including via a Bluetooth connection, e.g., Device A, Device B, ... Device E. Any of a range of alarm settings (FIG. 34) may be enabled, so that a user of the reader can be made aware of any signals falling outside a desired range.

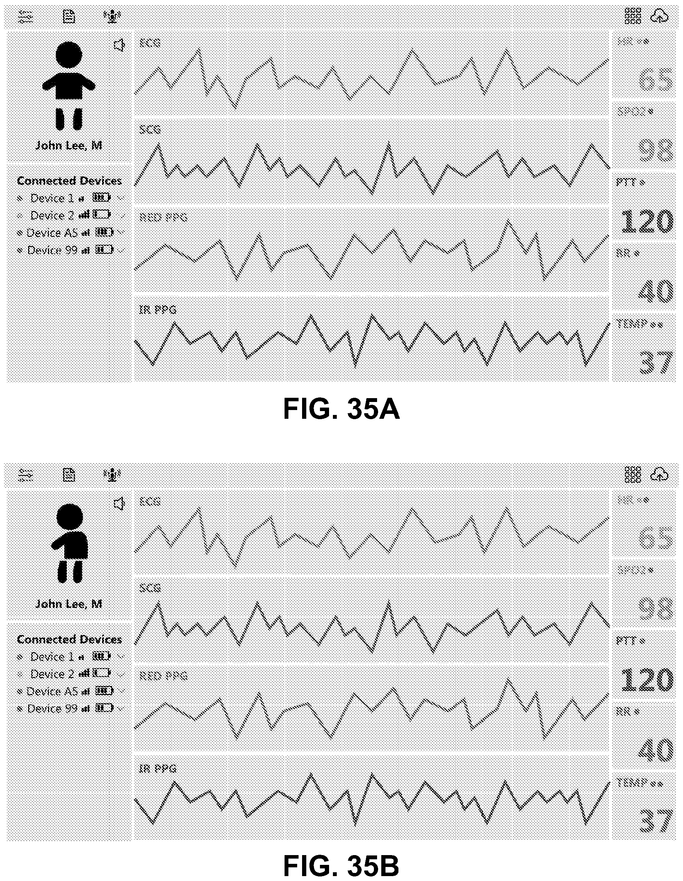

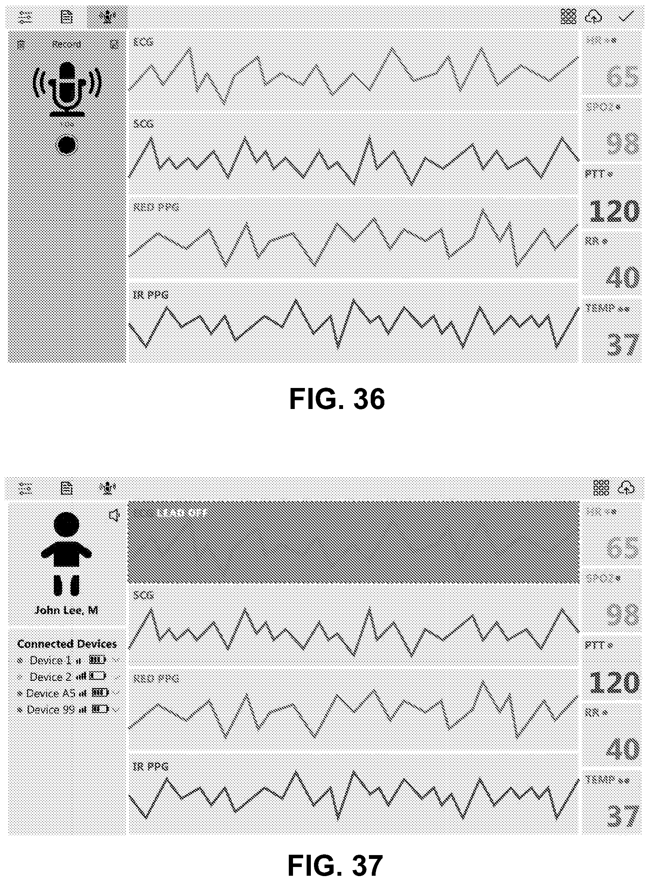

In addition, patient position can also be displayed, as shown in FIGS. 35A and 35B, which illustrate a patient on their back (FIG. 35A) versus more on their side (FIG. 35B). Notes are readily taken, either written, oral, or by picture/video, as shown in FIG. 36.

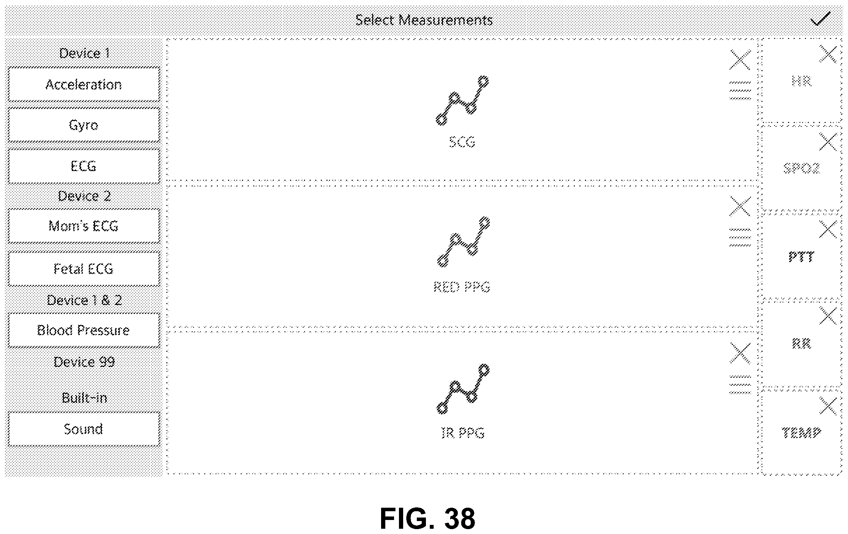

According to the invention, sensor malfunction occurs, such as a lead off condition, it can be rapidly conveyed, as shown in FIG. 37. Further sensor selection, control, and connection, including fetal and/or maternal, are compatible with the instant sensor networks, as shown in FIG. 38.

### TELEMEDICINE

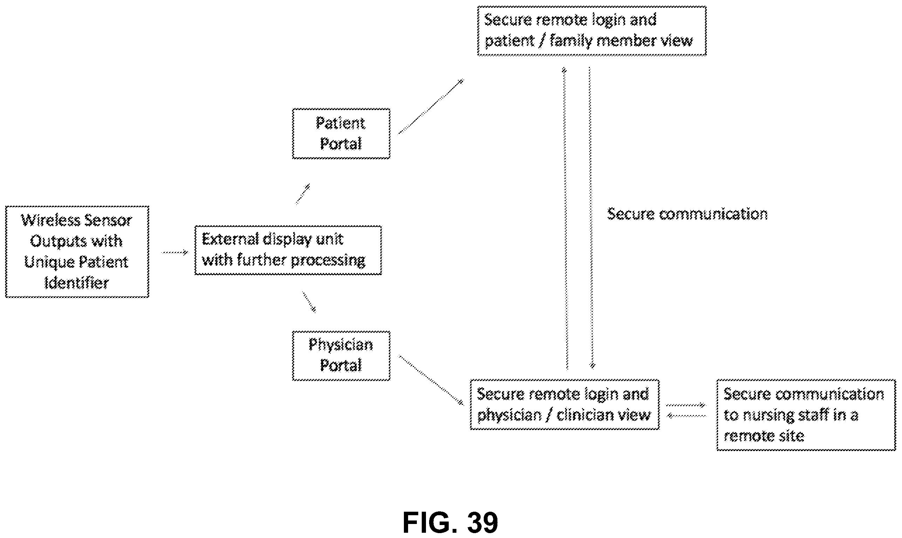

FIG. 39 illustrates the telemedical applications of the sensor network according to one embodiment of the invention. The sensor network has wireless outputs, with unique patient identifier information. The output is provided to a reader, which may have a display unit and processors for further processing the output data. The processed data may be provided to a physician/clinician and/or a patient portal, where secure remote logins ensure only authorized caregivers and persons have access to the data. There may be two-way communication between the patient/family member and caregiver. The caregiver may have two-way communication with a remote site where the patient is located, thereby ensuring appropriate orders are efficiently and rapidly conveyed to on-sight caregivers. Accordingly, the sensor outputs can be viewed by both the neonate's caregivers as well as their providers. The availability of skilled neonatologists trained in critical care is limited in developing countries or rural communities. The wearable sensors in this disclosure could also enable secure physician/clinician view allowing for eNICU care.

Referring to FIG. 40, a flowchart summary illustrating the benefits of measuring a physiological parameter with at least three different sensors is shown according to one embodiment of the invention. According to the embodiment of the sensor network, if all the three sensors obtain a similar reading that is out of a specified normal range for the parameter, a caregiver is notified with an alarm. If one sensor is out of a defined agreement range (e.g., +/-10%, or a user-determined range that may be relevant for the specific sensors being used and vital sign being measure), a notification is sent of an aberrant signal for that sensor so that a remedial action can be taken. Alternatively, if all the sensors are in agreement that the parameter is outside a "normal" range, the clinician may be notified so that immediate intervention/assessment is made. Of course, even while this is occurring, the sensors may be continuously in operation providing parameter measurements. For normal operating conditions, the dashed arrows reflect the sensor network simply continues normal operation.

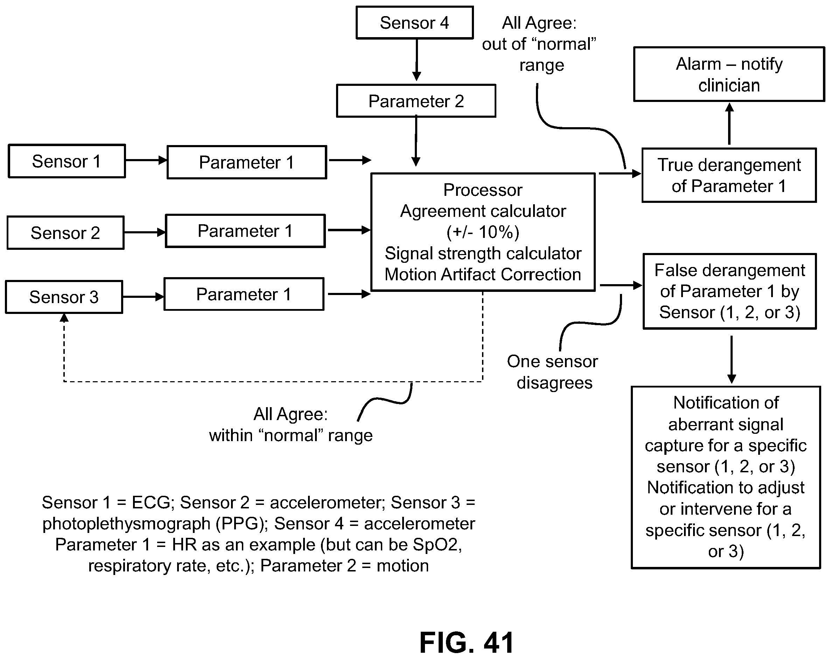

FIG. 41 shows a flow-chart summary similar to that of FIG. 40, but with an additional Sensor 4 accelerometer that is used to compensate and correct for motion artifacts, thereby further increasing sensor accuracy and reliability.

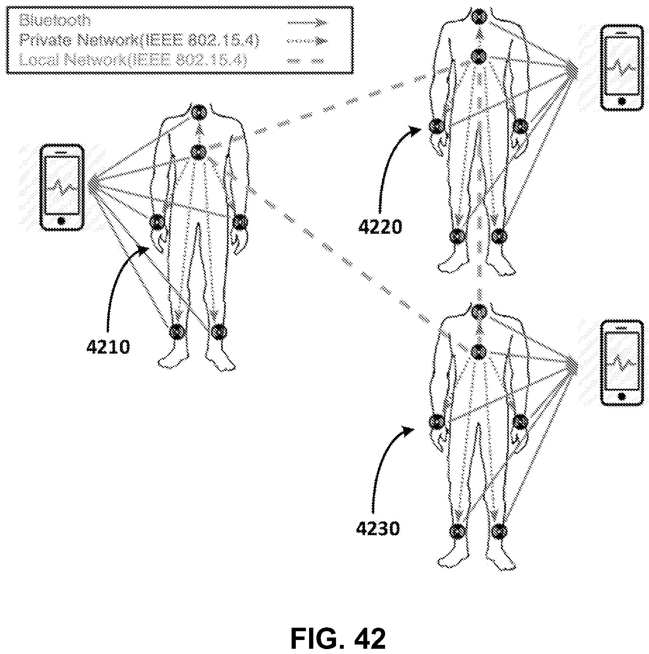

FIG. 42 is a schematic illustration of the wireless communication and a Bluetooth time synchronization to provide time-synchronized sensors according to one embodiment of the invention. In this exemplary embodiment, multiple sensor networks, e.g., 4210, 4220 and 4230, communicate with each other through, for example, local network. As disclosed above, each sensor network 4210, 4220 or 4230, has a plurality of sensor systems spatial-separately attached to torso regions and limb or appendage regions of a human subject and time-synchronized to each other and being in wireless communication with each other through, for example, a private network. Meanwhile, each sensor network 4210, 4220 or 4230 is in in wireless communication with a reader though Bluetooth. As such configuration, in addition to perform the measuring and/or monitoring functions as discussed for the human subject in which the sensor network is deployed, the sensor network 4210, 4220 or 4230 communicates with the others.

The foregoing description of the exemplary embodiments of the present invention has been presented only for the purposes of illustration and description and is not intended to be exhaustive or to limit the invention to the precise forms disclosed. Many modifications and variations are possible in light of the above teaching.

The embodiments were chosen and described in order to explain the principles of the invention and their practical application so as to activate others skilled in the art to utilize the invention and various embodiments and with various modifications as are suited to the particular use contemplated. Accordingly, the scope of the present invention is defined by the appended claims rather than the foregoing description and the exemplary embodiments described therein.

## Claims

1. A sensor network (100) for measuring physiological parameters of a mammal subject, comprising:
a plurality of spatially separated sensor systems (110/150, 210/ 250, 310/350) that is time-synchronized to each other, wherein each of the plurality of spatially separated sensor systems (110/150, 210/ 250, 310/350) is attached to a respective position of the mammal subject and comprises a sensor member for measuring at least one physiological parameter, a system on a chip, SOC, (124/164, 224/264, 324/364) having a microprocessor coupled to the sensor member for receiving data from the sensor member and processing the received data, and a transceiver (125/165, 195) coupled to the SoC (124/164, 224/264, 324/364) ; and
a microcontroller unit, MCU, (190) adapted in wireless communication with the plurality of spatially separated sensor systems (110/150, 210/ 250, 310/350) for wirelessly transmitting data to and from the plurality of spatially separated sensor systems (110/150, 210/ 250, 310/350),
wherein each two adjacent sensor systems (110/150, 210/ 250, 310/350) are spatially separated by a respective distance that is adjustable between a minimal distance and a maximal distance,
**characterized in that**
the transceiver coupled to the SoC (124/164, 224/264, 324/364) is adapted for wireless data transmission and wireless power harvesting, and
the plurality of spatially separated sensor systems (110/150, 210/ 250, 310/350) comprises a first sensor system (110, 210, 310) configured to attach to a central region (410) of the mammal subject and a second sensor system (150, 250, 350) configured to attach to an extremity region (420) of the mammal subject, wherein the central region (410) comprises one or more of a chest region, a neck region including a suprasternal notch area, and a head region including a forehead region or an anterior fontanelle region of the mammal subject, and wherein the extremity region (420) comprises one or more of a limb region, a foot region, a hand region, a toenail region, and a fingernail region of the mammal subject.

2. The sensor network of claim 1, wherein the sensor member (123, 223, 323) of the first sensor system (110, 210) comprises at least two electrodes (121, 122) spatially apart from each other for electrocardiogram, ECG, generation;
and/or, wherein the sensor member (163, 263, 363) of the second sensor system (150, 250, 350) comprises a photoplethysmogram, PPG, sensor comprising an optical source and an optical detector located within a sensor footprint.

3. The sensor network of claim 1 or 2, wherein the sensor member (123/163, 223/263, 323/363) further comprises one or more of:
an accelerometer for measuring at least one of a position and a movement;
an inertial measurement unit, IMU, for measuring at least one of a movement, a force, an angular rate, and an orientation;
a temperature sensor for measuring temperature.

4. The sensor network of claim 3, wherein the accelerometer or the IMU is used to measure at least one of seismocardiography, SCG, and a respiratory rate;
and/or, wherein the accelerometer or the IMU is used with a motion artifact module to identify a vital sign as subject to motion artifact and to correct of motion artifact.

5. The sensor network of claim 1 or 2, wherein the sensor member (123/163, 223/263, 323/363) comprises one or more of an ECG sensor, an EKG sensor, a pulse oximeter sensor, a temperature sensor, a blood pressure sensor, an accelerometer, or an acoustic sensor.

6. The sensor network of any one of claims 1-5, wherein the physiological parameters comprise one or more of heart activities including a stroke volume and ejection fraction, oxygenation level, temperature, skin temperature differentials, body movement, body position, breathing parameters, blood pressure, crying time, crying frequency, swallow count, swallow frequency, chest wall displacement, heart sounds, core body position, asynchronous limb motion, speaking, and biomechanical perturbation.

7. The sensor network of any one of claims 1-6, wherein the MCU (190) is configured to perform at least one function of:
receiving and processing measured data of the physiological parameters from the plurality of spatially separated sensor systems (110/150, 210/ 250, 310/350);
transmitting the processed data of the physiological parameters to at least one of a patient database, a cloud server, and a mobile device;
continuously multi-modal monitoring one or more critical parameters associating at least one vital sign; and notifying a practitioner or caregiver when a sensor aberrant signal output condition occurs; and
generating an alarm when an alarming vital sign reading condition in which the one or more of the critical parameters are out of pre-defined ranges occurs, and notifying a practitioner or caregiver of the alarm,
wherein the one or more critical parameters are one or more of the heart parameters, brain activities, temperature, body movements, respiratory parameters, oxygenation, vocalization parameters, swallow parameters, and blood pressure and blood flow.

8. The sensor network of any one of claims 1-7, wherein the MCU (190) is configured to further perform at least one function of:
assessing body pain of the mammal subject, based on the physiological parameters including a heart rate, heart rate variability, respiratory rate, respiratory effort, and crying time, and notifying a practitioner or caregiver where the pain is assessed;
assessing local blood perfusion of the mammal subject, based on a location specific pulse oximetry derived from peripheral oxygen saturation, SpO2, measured by pulse oximeters of the sensor systems placed on various locations of the body; and
detecting an apneic event when sudden decreases or cessation of the respiratory rate followed by compensatory increases in the heart rate and decreases in the SpO₂, and notifying a practitioner or caregiver when the apneic event occurs, and vibrating the sensor systems itself to trigger the mammal subject to change its position or awake from sleep; and
localizing anatomical pathology based on the physiological parameters measured by the plurality of spatially separated sensor systems (110/150, 210/ 250, 310/350) placed in differential locations.

9. The sensor network of any one of claims 1-8, wherein the MCU (190) comprises a mobile device for at least one of real-time display of the physiological parameters, recording of the physiological parameters, and alarm,
wherein the mobile device in bi-directionally wireless communication with the plurality of spatially separated sensor systems (110/150, 210/ 250, 310/350);
and/or, wherein the mobile device is in wireless communication with the patient database;
and/or, wherein the mobile device is a hand-held device or a portable device having a graphical user interface to display the plurality of physiological parameters.

10. The sensor network of any one of claims 1-9, further comprising a power unit for wirelessly powering the sensor systems.

11. The sensor network of claim 10, being capable of operating wirelessly for a period of at least 24 hours without a source of external power.

12. The sensor network of any one of claims 1-11, wherein at least one of the plurality of spatially separated sensor systems (110/150, 210/250, 310/350) further comprises an actuator configured to generate a force for providing a stimulus to the mammal subject when a pre-defined trigger signal is detected;
wherein the actuator is one or more of an electromechanical motor, a heater, and an electrical stimulator; and
wherein the stimulus comprises gentle vibrations for soothing the mammal subj ect.

13. The sensor network of any one of claims 1-12, wherein each of the plurality of spatially separated sensor systems (110/150, 210/ 250, 310/350) further comprises:
a plurality of flexible and stretchable interconnects electrically connecting to a plurality of electronic components including the sensor member, the SoC (124/164, 224/264, 324/364) and the transceiver; and
an elastomeric encapsulation layer surrounding the electronic components and the plurality of flexible and stretchable interconnects to form a tissue-facing surface and an environment-facing surface, wherein the tissue-facing surface is configured to conform to a skin surface of the mammal subject.

14. The sensor network of claim 13, wherein the transceiver comprises a magnetic loop antenna configured to allow simultaneous wireless data transmission and wireless power harvesting through a single link.

15. The sensor network of claim 14, wherein the electronic components of each of the plurality of spatially separated sensor systems (110/150, 210/250, 310/350) further comprises a battery (205) for provide power to said sensor system, and the elastomeric encapsulation layer is configured to electrically isolate the battery (205) from the mammal subject during use,
wherein the battery (205) is a rechargeable battery operably recharged with wireless recharging, and
wherein the encapsulation layer comprises a flame retardant material.

16. The sensor network of claim 15, wherein the electronic components of each of the plurality of spatially separated sensor systems (110/150, 210/250, 310/350) further comprises a failure prevention element that is a short-circuit protection component or a battery circuit to avoid battery explosion.

## Patentansprüche

1. Sensornetzwerk (100) zur Messung physiologischer Parameter eines Säugers, umfassend:
eine Pluralität von räumlich getrennten Sensorsystemen (110/150, 210/250, 310/350), die zueinander zeitsynchronisiert ist, wobei jedes der Pluralität von räumlich getrennten Sensorsystemen (110/150, 210/250, 310/350) an einer jeweiligen Position des Säugers angebracht ist und
ein Sensorglied zum Messen von mindestens einem physiologischen Parameter, ein System auf einem Chip, SoC, (124/164, 224/264, 324/364) mit einem Mikroprozessor, der mit dem Sensorglied gekoppelt ist, zum Empfangen von Daten von dem Sensorglied und zum Verarbeiten der empfangenen Daten, und einen Transceiver (125/165, 195), der mit dem SoC (124/164, 224/264, 324/364) gekoppelt ist umfasst; und
eine Mikrocontrollereinheit, MCU (190), die in drahtloser Kommunikation mit der Pluralität von räumlich getrennten Sensorsystemen (110/150, 210/250, 310/350) zum drahtlosen Übertragen von Daten zu und von der Pluralität von räumlich getrennten Sensorsystemen (110/150, 210/250, 310/350) adaptiert ist,
wobei jeweils zwei benachbarte Sensorsysteme (110/150, 210/250, 310/350) räumlich durch einen jeweiligen Abstand getrennt sind, der zwischen einem minimalen Abstand und einem maximalen Abstand einstellbar ist, **dadurch gekennzeichnet, dass**
der mit dem SoC (124/164, 224/264, 324/364) gekoppelte Transceiver zur drahtlosen Datenübertragung und zur drahtlosen Energiegewinnung adaptiert ist, und
die Pluralität der räumlich getrennten Sensorsysteme (110/150, 210/250, 310/350) ein erstes Sensorsystem (110, 210, 310) umfasst, das so konfiguriert ist, dass es an einem zentralen Bereich (410) des Säugers angebracht wird, und
ein zweites Sensorsystem (150, 250, 350), das so konfiguriert ist, dass es an einem Extremitätenbereich (420) des Säugers angebracht wird,
wobei der zentrale Bereich (410) einen oder mehrere aus einem Brustbereich, einem Halsbereich einschließlich eines suprasternalen Kerbenbereichs und einem Kopfbereich einschließlich eines Stirnbereichs oder eines vorderen Fontanellenbereichs des Säugers umfasst, und
wobei der Extremitätenbereich (420) einen oder mehrere aus einem Gliedmaßenbereich, einem Fußbereich, einem Handbereich, einem Zehennagelbereich und einem Fingernagelbereich des Säugers umfasst.

2. Sensornetzwerk nach Anspruch 1, wobei das Sensorglied (123, 223, 323) des ersten Sensorsystems (110, 210) mindestens zwei räumlich voneinander getrennte Elektroden (121, 122) zur Erzeugung eines Elektrokardiogramms (ECG) umfasst; und/oder,
wobei das Sensorglied (163, 263, 363) des zweiten Sensorsystems (150, 250, 350) einen Photoplethysmogramm-, PPG-, Sensor umfasst, der eine optische Quelle und einen optischen Detektor umfasst, die innerhalb eines Sensorerfassungsbereichs lokalisiert sind.

3. Das Sensornetzwerk nach Anspruch 1 oder 2, wobei das Sensorglied (123/163, 223/263, 323/363) ferner eines oder mehrere von:
einem Beschleunigungsmesser zum Messen mindestens einer Position und einer Bewegung;
einer Inertialmesseinheit (IMU) zum Messen von mindestens einer von Bewegung, einer Kraft, einer Winkelrate und einer Orientierung;
einem Temperatursensor zum Messen der Temperatur umfasst.

4. Sensornetzwerk nach Anspruch 3, wobei der Beschleunigungsmesser oder die IMU verwendet wird, um mindestens eines von Seismokardiographie, SCG, und einer Respirationsfrequenz zu messen; und/oder,
wobei der Beschleunigungsmesser oder die IMU mit einem Bewegungsartefaktmodul verwendet wird, um ein Vitalzeichen als von einem Bewegungsartefakt betroffen zu identifizieren und das Bewegungsartefakt zu korrigieren.

5. Sensornetzwerk nach Anspruch 1 oder 2, wobei das Sensorglied (123/163, 223/263, 323/363) einen oder mehrere von einem ECG-Sensor, einem EKG-Sensor, einem Pulsoximetersensor, einem Temperatursensor, einem Blutdrucksensor, einem Beschleunigungsmesser oder einem akustischen Sensor umfasst.

6. Das Sensornetzwerk nach einem der Ansprüche 1-5, wobei die physiologischen Parameter eine oder mehrere der Herzaktivitäten umfassen, einschließlich Schlagvolumen und Ejektionsfraktion, Sauerstoffgehalt, Temperatur, Hauttemperaturdifferenzen, Körperbewegung, Körperposition, Atmungsparameter, Blutdruck, Schreizeit, Schreihäufigkeit, Schluckanzahl, Schluckhäufigkeit, Brustwandverschiebung, Herztöne, Zentralkörperposition, asynchrone Gliedmaßenbewegung, Sprechen und biomechanische Perturbation.

7. Sensornetzwerk nach einem der Ansprüche 1 bis 6, wobei die MCU (190) so konfiguriert ist, dass sie mindestens eine der folgenden Funktionen ausführt:
Empfangen und Verarbeiten von Messdaten der physiologischen Parameter von der Pluralität der räumlich getrennten Sensorsystemen (110/150, 210/250, 310/350);
Übertragen der verarbeiteten Daten der physiologischen Parameter an mindestens eine Patientendatenbank, einen Cloud-Server oder ein Mobilgerät;
kontinuierliches multimodales Überwachen eines oder mehrerer kritischer Parameter, die mit mindestens einem Vitalzeichen assoziiert sind; und
Benachrichtigen eines Praktikers oder einer Pflegeperson, wenn ein aberranter Signalausgabezustand des Sensors auftritt; und
Erzeugen eines Alarms, wenn ein alarmierender Vitalzeichen-Ablesezustand auftritt, bei dem der eine oder mehrere der kritischen Parameter außerhalb vordefinierter Bereiche liegen, und Benachrichtigen eines Praktikers oder einer Pflegeperson über den Alarm,
wobei der eine oder mehrere kritische Parameter einer oder mehrere von Herzparameter, Gehirnaktivitäten, Temperatur, Körperbewegungen, Atmungsparameter, Sauerstoffversorgung, Vokalisationsparameter, Schluckparameter sowie Blutdruck und Blutfluss sind.

8. Sensornetzwerk nach einem der Ansprüche 1-7, wobei die MCU (190) so konfiguriert ist, dass sie ferner mindestens eine der folgenden Funktionen ausführt:
Beurteilen des Körperschmerzes des Säugers, basierend auf den physiologischen Parametern, einschließlich einer Herzfrequenz, Herzfrequenzvariabilität, Respirationfrequenz, respiratorischen Anstrengung und Schreizeit, und Benachrichtigen eines Praktikers oder einer Pflegeperson, wo der Schmerz beurteilt wird;
Beurteilen der lokalen Blutperfusion des Säugers basierend auf einer ortsspezifischen Pulsoximetrie, die von der peripheren Sauerstoffsättigung, SpO2, abgeleitet wird, die von Pulsoximetern der Sensorsysteme gemessen wird, die an verschiedenen Stellen des Körpers platziert sind; und
Detektieren eines Apnoe-Ereignisses, wenn plötzliche Abnahmen oder Beendigung der Respirationsrate gefolgt von einem kompensatorischen Anstieg der Herzrate und Abnahmen des SpO2 auftritt, und Benachrichtigen eines Praktikers oder einer Pflegeperson, wenn das Apnoe-Ereignis auftritt, und Vibrieren der Sensorsysteme selbst, um den Säuger zu veranlassen, seine Position zu ändern oder aus dem Schlaf zu erwachen; und
Lokalisieren von anatomischer Pathologie basierend auf den physiologischen Parametern, die von der Pluralität der räumlich getrennten Sensorsysteme (110/150, 210/250, 310/350) gemessen werden, die an unterschiedlichen Orten platziert sind.

9. Sensornetzwerk nach einem der Ansprüche 1-8, wobei die MCU (190) ein Mobilgerät für mindestens eine von Echtzeitanzeige der physiologischen Parameter, Aufzeichnung der physiologischen Parameter und Alarm umfasst, wobei das Mobilgerät in bidirektionaler drahtloser Kommunikation mit der Pluralität der räumlich getrennten Sensorsysteme (110/150, 210/250, 310/350) steht; und/oder
wobei das Mobilgerät in drahtloser Kommunikation mit der Patientendatenbank steht; und/oder,
wobei das Mobilgerät ein Handheld-Gerät oder ein tragbares Gerät mit einer grafischen Benutzeroberfläche zur Anzeige der Pluralität der physiologischen Parameter ist.

10. Das Sensornetz nach einem der Ansprüche 1 bis 9 ferner umfassend eine Stromversorgungseinheit für die drahtlose Stromversorgung der Sensorsysteme.

11. Das Sensornetz nach Anspruch 10, das in der Lage ist, für eine Periode von mindestens 24 Stunden drahtlos ohne externe Energiequelle zu operieren.

12. Sensornetzwerk nach einem der Ansprüche 1 bis 11, wobei mindestens eines der Pluralität der räumlich getrennten Sensorsysteme (10/150, 210/250, 310/350) ferner einen Aktuator umfasst, der so konfiguriert ist, dass er eine Kraft erzeugt, um dem Säuger einen Stimulus zu vermitteln, wenn ein vordefiniertes Triggersignal detektiert wird;
wobei der Aktuator eines oder mehrere aus einem elektromechanischen Motor, einem Heizer und einem elektrischen Stimulator ist; und
wobei der Stimulus sanfte Vibrationen umfasst, um den Säuger zu beruhigen.

13. Sensornetzwerk nach einem der Ansprüche 1 bis 12, wobei jedes der Pluralität der räumlich getrennten Sensorsysteme (110/150, 210/250, 310/350) ferner umfasst:
eine Pluralität von flexiblen und dehnbaren Interkonnektoren, die elektrisch mit einer Pluralität von elektronischen Komponenten verbunden sind, einschließlich des Sensorglieds, des SoC (124/164, 224/264, 324/364) und des Transceivers; und
eine elastomere Verkapselungsschicht, die die elektronischen Komponenten und die Pluralität der flexiblen und dehnbaren Verbindungen umgibt, um eine dem Gewebe zugewandte Oberfläche und eine der Umgebung zugewandte Oberfläche zu bilden, wobei die dem Gewebe zugewandte Oberfläche so konfiguriert ist, dass sie sich an eine Hautoberfläche des Säugers anpasst.

14. Sensornetzwerk nach Anspruch 13, wobei der Transceiver eine magnetische Rahmenantenne umfasst, die so konfiguriert ist, dass sie eine gleichzeitige drahtlose Datenübertragung und drahtlose Energiegewinnung über eine einzige Verbindung ermöglicht.

15. Sensornetzwerk nach Anspruch 14, wobei die elektronischen Komponenten jedes der Pluralität der räumlich getrennten Sensorsysteme (110/150, 210/250, 310/350) ferner eine Batterie (205) umfassen, um das Sensorsystem mit Energie zu versorgen, und die elastomere Verkapselungsschicht so konfiguriert ist, dass sie die Batterie (205) während der Verwendung von dem Säuger elektrisch isoliert,
wobei die Batterie (205) eine wiederaufladbare Batterie ist, die durch drahtloses Aufladen wiederaufladbar ist, und
wobei die Verkapselungsschicht ein flammhemmendes Material umfasst.

16. Sensornetzwerk nach Anspruch 15, wobei die elektronischen Komponenten jedes der Pluralität der räumlich getrennten Sensorsysteme (110/150, 210/250, 310/350) ferner ein Element zur Fehlervermeidung umfassen, bei dem es sich um eine Kurzschlussschutzkomponente oder eine Batterieschaltung zur Vermeidung einer Batterieexplosion handelt.

## Revendications

1. Un réseau de capteurs (100) pour mesurer des paramètres physiologiques d'un sujet mammifère, comprenant :
une pluralité de systèmes de capteurs séparés dans l'espace (110/150, 210/ 250, 310/350) qui sont synchronisés dans le temps les uns par rapport aux autres, dans lequel chacun de la pluralité de systèmes de capteurs séparés dans l'espace (110/150, 210/ 250, 310/350) est attaché à une position respective du sujet mammifère et comprend un élément capteur pour mesurer au moins un paramètre physiologique, un système sur puce, SoC, (124/164, 224/264, 324/364) doté d'un microprocesseur couplé à l'élément capteur pour recevoir des données de l'élément capteur et traiter les données reçues, et un émetteur-récepteur (125/165, 195) couplé au SoC (124/164, 224/264, 324/364); et
une unité de microcontrôleur, MCU, (190) adaptée à la communication sans fil avec la pluralité de systèmes de capteurs séparés dans l'espace (110/150, 210/250, 310/350) pour transmettre sans fil des données vers et depuis la pluralité de systèmes de capteurs séparés dans l'espace (110/150, 210/250, 310/350),
dans lequel, pour chaque ensemble de deux systèmes de capteurs adjacents (110/150, 210/250, 310/350), les deux systèmes sont séparés dans l'espace par une distance respective réglable entre une distance minimale et une distance maximale, **caractérisé en ce que**
l'émetteur-récepteur couplé au SoC (124/164, 224/264, 324/364) est adapté à la transmission de données sans fil et à la collecte d'énergie sans fil, et
la pluralité de systèmes de capteurs séparés dans l'espace (110/150, 210/250, 310/350) comprend un premier système de capteur (110, 210, 310) configuré pour se fixer à une région centrale (410) du sujet mammifère et un second système de capteur (150, 250, 350) configuré pour se fixer à une région d'extrémité (420) du sujet mammifère, dans lequel la région centrale (410) comprend une ou plusieurs des régions suivantes : une région thoracique, une région du cou, y compris une région de l'échancrure suprasternale, et une région de la tête, y compris une région du front ou une région de la fontanelle antérieure du sujet mammifère, et dans lequel la région d'extrémité (420) comprend une ou plusieurs des régions suivantes : une région des membres, une région du pied, une région de la main, une région de l'ongle de pied et une région de l'ongle de main du sujet mammifère.

2. Le réseau de capteurs de la revendication 1, dans lequel l'élément capteur (123, 223, 323) du premier système de capteurs (110, 210) comprend au moins deux électrodes (121, 122) espacées l'une de l'autre pour la génération d'un électrocardiogramme, ECG;
et/ou, dans lequel l'élément capteur (163, 263, 363) du deuxième système de capteurs (150, 250, 350) comprend un capteur de photopléthysmogramme, PPG, comprenant une source optique et un détecteur optique situés dans l'empreinte du capteur.

3. Le réseau de capteurs de la revendication 1 ou 2, dans lequel l'élément de capteur (123/163, 223/263, 323/363) comprend en outre un ou plusieurs des éléments suivants:
un accéléromètre pour mesurer une position et/ou un mouvement;
une unité de mesure inertielle, IMU, pour mesurer au moins l'une des valeurs suivantes: un mouvement, une force, une vitesse angulaire et une orientation;
un capteur de température pour mesurer la température.

4. Le réseau de capteurs de la revendication 3, dans lequel l'accéléromètre ou l'IMU est utilisé pour mesurer au moins l'un des paramètres suivants: sismocardiographie, SCG, et fréquence respiratoire;
et/ou, dans lequel l'accéléromètre ou l'IMU est utilisé avec un module d'artefact de mouvement pour identifier un signe vital comme étant sujet à un artefact de mouvement et pour corriger l'artefact de mouvement.

5. Le réseau de capteurs de la revendication 1 ou 2, dans lequel l'élément capteur (123/163, 223/263, 323/363) comprend un ou plusieurs des éléments suivants : un capteur ECG, un capteur EKG, un capteur d'oxymètre de pouls, un capteur de température, un capteur de pression sanguine, un accéléromètre ou un capteur acoustique.

6. Le réseau de capteurs de l'une des revendications 1 à 5, dans lequel les paramètres physiologiques comprennent un ou plusieurs des éléments suivants : des activités cardiaques, y compris un volume d'apoplexie et une fraction d'éjection, un niveau d'oxygénation, une température, des différentiels de température de la peau, des mouvements du corps, une position du corps, des paramètres respiratoires, une pression artérielle, une durée des pleurs, une fréquence des pleurs, un nombre de déglutitions, une fréquence des déglutitions, un déplacement de la paroi thoracique, bruits du coeur, une position centrale du corps, un mouvement asynchrone des membres, une élocution et une perturbation biomécanique.

7. Le réseau de capteurs de l'une des revendications 1 à 6, dans lequel la MCU (190) est configurée pour exécuter au moins l'une des fonctions suivantes :
réception et traitement des données mesurées des paramètres physiologiques provenant de la pluralité de systèmes de capteurs séparés dans l'espace (110/150, 210/250, 310/350);
transmission des données traitées des paramètres physiologiques à au moins l'un des récepteurs suivants: une base de données patient, un serveur en nuage et un dispositif mobile;
surveillance multimodale continue d'un ou de plusieurs des paramètres critiques associant au moins un signe vital; et notification à un praticien ou à un soignant lorsqu'un signal de sortie aberrant du capteur se produit; et
génération d'une alarme lorsqu'une condition de lecture de signe vital alarmante dans laquelle un ou plusieurs des paramètres critiques sont en dehors des plages prédéfinies se produit, et notification de l'alarme à un praticien ou à un soignant,
dans lequel le ou les paramètres critiques sont un ou plusieurs paramètres choisi(s) de : des paramètres cardiaques, des activités cérébrales, une température, des mouvements corporels, des paramètres respiratoires, une oxygénation, des paramètres de vocalisation, des paramètres de déglutition, une pression artérielle et un débit sanguin.

8. Le réseau de capteurs de l'une des revendications 1 à 7, dans lequel la MCU (190) est configurée pour exécuter au moins l'une des fonctions suivantes :
évaluation de la douleur corporelle du sujet mammifère, sur la base des paramètres physiologiques comprenant une fréquence cardiaque, une variabilité de la fréquence cardiaque, une fréquence respiratoire, un effort respiratoire et un temps de pleurs, et notification à un praticien ou un soignant où la douleur est évaluée;
évaluation de la perfusion sanguine locale du sujet mammifère, sur la base d'une oxymétrie de pouls spécifique au lieu, dérivée de la saturation périphérique en oxygène, SpO2, mesurée par les oxymètres de pouls des systèmes de capteurs placés à différents endroits du corps; et
détection d'un événement apnéique lorsque des diminutions soudaines ou un arrêt de la fréquence respiratoire sont suivis par des augmentations compensatoires de la fréquence cardiaque et des diminutions de la SpO2, et notification à un praticien ou un soignant lorsque l'événement apnéique se produit, et vibration des systèmes de capteurs eux-mêmes pour déclencher le changement de position du sujet mammifère ou son réveil du sommeil; et
localisation d'une pathologie anatomique sur la base des paramètres physiologiques mesurés par la pluralité de systèmes de capteurs séparés dans l'espace (110/150, 210/250, 310/350) placés à des endroits différentiels.

9. Le réseau de capteurs de l'une des revendications 1 à 8, dans lequel la MCU (190) comprend un dispositif mobile pour au moins l'une des actions suivantes: affichage en temps réel des paramètres physiologiques, enregistrement des paramètres physiologiques et alarme,
dans lequel le dispositif mobile est en communication bidirectionnelle sans fil avec la pluralité de systèmes de capteurs séparés dans l'espace (110/150, 210/250, 310/350);
et/ou, dans lequel le dispositif mobile est en communication sans fil avec la base de données du patient;
et/ou, dans lequel le dispositif mobile est un dispositif à main ou un dispositif portable doté d'une interface utilisateur graphique permettant d'afficher la pluralité de paramètres physiologiques.

10. Le réseau de capteurs de l'une des revendications 1 à 9, comprenant en outre une unité d'alimentation pour alimenter sans fil les systèmes de capteurs.

11. Le réseau de capteurs de la revendication 10, capable de fonctionner sans fil pendant une période d'au moins 24 heures sans source d'énergie externe.

12. Le réseau de capteurs de l'une des revendications 1 à 11, dans lequel au moins un des systèmes de capteurs séparés dans l'espace (110/150, 210/250, 310/350) comprend en outre un actionneur configuré pour générer une force afin de fournir un stimulus au sujet mammifère lorsqu'un signal de déclenchement prédéfini est détecté;
dans lequel l'actionneur est un ou plusieurs des éléments suivants: un moteur électromécanique, un élément chauffant et un stimulateur électrique; et
dans lequel le stimulus comprend de légères vibrations pour apaiser le mammifère.

13. Le réseau de capteurs de l'une des revendications 1 à 12, dans lequel chacun des systèmes de capteurs séparés dans l'espace (110/150, 210/250, 310/350) comprend en outre :
une pluralité d'interconnexions flexibles et extensibles reliées électriquement à une pluralité de composants électroniques, y compris l'élément capteur, le SoC (124/164, 224/264, 324/364) et l'émetteur-récepteur; et
une couche d'encapsulation élastomère entourant les composants électroniques et la pluralité d'interconnexions flexibles et extensibles pour former une surface orientée vers les tissus et une surface orientée vers l'environnement, la surface orientée vers les tissus étant configurée pour s'adapter à la surface de la peau du sujet mammifère.

14. Le réseau de capteurs de la revendication 13, dans lequel l'émetteur-récepteur comprend une antenne à boucle magnétique configurée pour permettre la transmission simultanée de données sans fil et la collecte d'énergie sans fil par le biais d'une seule liaison.

15. Le réseau de capteurs de la revendication 14, dans lequel les composants électroniques de chacun des systèmes de capteurs séparés dans l'espace (110/150, 210/250, 310/350) comprennent en outre une batterie (205) pour alimenter ledit système de capteurs, et la couche d'encapsulation élastomère est configurée pour isoler électriquement la batterie (205) du sujet mammifère pendant l'utilisation,
dans laquelle la batterie (205) est une batterie rechargeable qui peut être rechargée sans fil, et
la couche d'encapsulation comprend un matériau ignifuge.

16. Le réseau de capteurs de la revendication 15, dans lequel les composants électroniques de chacun des systèmes de capteurs séparés dans l'espace (110/150, 210/250, 310/350) comprennent en outre un élément de prévention des défaillances qui est un composant de protection contre les courts-circuits ou un circuit de batterie afin d'éviter l'explosion de la batterie.
